# EUROPÄISCHE PATENTSCHRIFT

(11) **EP 3 213 069 B1**
(45) Veröffentlichungstag und Bekanntmachung des Hinweises auf die Patenterteilung: **12.12.2018**
(21) Anmeldenummer: 15775435.9
(22) Anmeldetag: 05.10.2015
(51) Int. Cl.: G01N 33/493, A61B 10/00, E03D 11/02

(54) **VORRICHTUNG ZUR ANALYSE VON URIN**
APPARATUS FOR ANALYZING URINE
DISPOSITIF POUR L'ANALYSE D'URINE

(30) Priorität: 31.10.2014 DE 102014115914
(43) Veröffentlichungstag der Anmeldung: 06.09.2017
(60) Teilanmeldung: 18169356.5
(73) Patentinhaber: Duravit Aktiengesellschaft, 78132 Hornberg (DE)
(72) Erfinder: SPANGENBERG, Bernd, 77654 Offenburg (DE); JANSEN, Dirk, 77797 Ohlsbach (DE); KAISER, Bernd, 79346 Endingen (DE)
(74) Vertreter: Lindner Blaumeier Patent- und Rechtsanwälte
(86) Internationale Anmeldenummer: PCT/EP2015/072908
(87) Internationale Veröffentlichungsnummer: WO 2016/066372

(56) Entgegenhaltungen:
- WO-A2-2006/065705
- DE-A1-102010 061 035
- US-A- 4 943 416
- US-A- 5 087 556
- US-A- 5 271 895
- US-A- 5 846 490
- US-A1- 2012 329 170

## Beschreibung

Die Erfindung betrifft eine Vorrichtung zur Analyse von Urin und eine eine solche Vorrichtung umfassende Sanitäreinrichtung.

Vorrichtungen zur Analyse von Urin sowie gegebenenfalls anderer körpereigener Fluide sind bekannt und dienen im Allgemeinen dazu, eine bestimmte Urinmenge im Hinblick auf ihre chemische bzw. anteilsmäßige Zusammensetzung zu analysieren. Üblicherweise werden hierzu Urinteststreifen verwendet, welche mit einer bestimmten Urinmenge kontaktiert werden. Die Auswertung bzw. Beobachtung einer Veränderung eines optisch erfassbaren Parameters, d. h. insbesondere eines Farbumschlags, eines urinteststreifenseitigen Analysebereichs lässt dabei Rückschlüsse auf die chemische bzw. anteilsmäßige Zusammensetzung des Urins zu.

Bisher ist es üblich, entsprechende Urinteststreifen manuell mit einer bestimmten Urinmenge zu benetzen, was z. B. durch Eintauchen eines Urinteststreifens in einen Probebehälter realisiert wird. Die mit Urin benetzten Urinteststreifen werden nachfolgend in einem Spektrometer begutachtet. Dies stellt ein vergleichsweise aufwändiges und somit verbesserungswürdiges Vorgehen zur Realisierung einer Analyse von Urin dar.

Aus DE 10 2010 061 035 A1 ist eine intelligente Toilette beschrieben, die eine Urinauffangvorrichtung aufweist, über die Urin zu einer Analyseeinrichtung geführt werden kann. Der Urin wird in eine lichtdurchlässige Küvette eingebracht, aus der er auch wieder entnommen werden kann, wobei zum Ein- und Ausbringen eine Pumpeinrichtung vorgesehen ist. Vorgesehen ist des Weiteren eine Lichtquelle, mit der Licht auf die Küvette gestrahlt werden kann, sowie wenigstens ein Lichtsensor, wobei Lichtquelle und Lichtsensor derart zueinander ausgerichtet sind, dass eine Absorptions- und eine Streulichtmessung an der Urinprobe durchführbar ist. Des Weiteren ist ein Farbindikator-Ver- und Entsorgungssystem zum Ein- und Ausbringen eines Farbindikators in die und aus der Küvette sowie zum Entsorgen des Farbindikators nach dessen Nutzung vorgesehen. Durch die lichtoptische Messung ist die Urinprobe auswertbar, worüber Schlussfolgerungen auf den Gesundheitszustand des Toilettenbenutzers gezogen werden können.

Der Erfindung liegt damit die Aufgabe zugrunde, eine demgegenüber verbesserte Vorrichtung zur Analyse von Urin anzugeben.

Die Aufgabe wird erfindungsgemäß durch eine Vorrichtung zur Analyse von Urin gelöst, welche Vorrichtung zumindest umfasst:
eine Zuführ- und Abführeinrichtung, welche zur Zuführung einer bestimmten Urinmenge in eine wenigstens einen Analysebereich aufweisende Analysekammer eines Urinteststreifens und zur Abführung einer bestimmten Urinmenge aus einer wenigstens einen Analysebereich aufweisenden Analysekammer eines Urinteststreifens eingerichtet ist, wobei die Zuführ- und Abführeinrichtung wenigstens ein bewegbar gelagertes Zuführ- und/oder Abführelement zum Zuführen einer bestimmten Urinmenge in einen Zuführbereich der Analysekammer des Urinteststreifens und/oder zum Abführen einer bestimmten Urinmenge aus einem Abführbereich der Analysekammer des Urinteststreifens aufweist, und eine Erfassungseinrichtung, welche zur Erfassung einer zumindest abschnittsweisen Änderung wenigstens eines optisch erfassbaren Parameters, welcher sich in Abhängigkeit der Zusammensetzung einer diesen kontaktierenden Urinmenge optisch erfassbar verändert, des oder eines entsprechenden Analysebereichs des oder eines entsprechenden Urinteststreifens sowie zur Erzeugung einer Erfassungsinformation, welche wenigstens einen optisch erfassten Parameter des oder eines entsprechenden Analysebereichs oder eine Änderung eines solchen beschreibt, eingerichtet ist.

Die erfindungsgemäße Vorrichtung zur Analyse von Urin, im Weiteren kurz als Vorrichtung bezeichnet, umfasst als wesentliche Bestandteile sonach eine Zuführ- und Abführeinrichtung und eine Erfassungseinrichtung. Selbstverständlich kann die Vorrichtung auch mehrere Zuführ- und Abführeinrichtungen bzw. mehrere Erfassungseinrichtungen umfassen.

Die Zuführ- und Abführeinrichtung dient der Zuführung einer bestimmten Urinmenge in eine wenigstens einen Analysebereich aufweisende Analysekammer eines Urinteststreifens und der Abführung einer bestimmten Urinmenge aus einer wenigstens einen Analysebereich aufweisenden Analysekammer eines Urinteststreifens. Entsprechend weist die Zuführ- und Abführeinrichtung wenigstens ein bewegbar gelagertes Zuführ- und/oder Abführelement zum Zuführen einer bestimmten Urinmenge in einen Zuführbereich der Analysekammer des Urinteststreifens und/oder zum Abführen einer bestimmten Urinmenge aus einem Abführbereich der Analysekammer des Urinteststreifens auf. Über die Zuführ- und Abführeinrichtung respektive das wenigstens eine dieser zugehörige Zuführ- und/oder Abführelement lässt sich sonach eine Zu- und/oder Abführung einer bestimmten Urinmenge, allgemein einer bestimmten Fluidmenge, in einen entsprechenden Zuführbereich bzw. aus einem entsprechenden Abführbereich und somit in eine bzw. aus einer urinteststreifenseitigen Analysekammer, in welcher ein entsprechender Analysebereich vorhanden ist, realisieren.

Wenngleich die Zu- und Abführung einer bestimmten Urinmenge grundsätzlich über ein einziges Zuführ- und Abführelement möglich ist, welches sowohl der Zuführung einer bestimmten Urinmenge in einen entsprechenden Zuführbereich als auch der Abführung einer bestimmten Urinmenge aus einem entsprechenden Abführbereich dient, weist die Zuführ- und Abführeinrichtung typischerweise wenigstens ein separates Zuführelement zum Zuführen einer bestimmten Urinmenge in einen entsprechenden Zuführbereich und wenigstens ein separates Abführelement zum Abführen einer bestimmten Urinmenge aus einem entsprechenden Abführbereich auf.

Die bewegbare Lagerung eines entsprechenden Zuführ- und/oder Abführelements ermöglicht gezielte Bewegungen dieses auf entsprechende urinteststreifenseitige Zuführ- und Abführbereiche zu respektive von entsprechenden urinteststreifenseitige Zuführ- und Abführbereichen weg. Die Bewegung eines entsprechenden Zuführ- und/oder Abführelements ist typischerweise zwischen zwei Bewegungsendpunkten möglich. Dabei definiert sich ein erster Bewegungsendpunkt dadurch, dass in diesem eine Zuführung einer bestimmten Urinmenge in einen entsprechenden urinteststreifenseitigen Zuführbereich bzw. eine Abführung einer bestimmten Urinmenge aus einem entsprechenden urinteststreifenseitigen Abführbereich möglich ist. Ein zweiter Bewegungsendpunkt definiert sich entsprechend dadurch, dass in diesem eine Zuführung einer bestimmten Urinmenge in einen entsprechenden urinteststreifenseitigen Zuführbereich bzw. eine Abführung einer bestimmten Urinmenge aus einem entsprechenden urinteststreifenseitigen Abführbereich nicht möglich ist.

Die Bewegung eines entsprechenden Zuführ- und/oder Abführelements erfolgt typischerweise entlang einer definierten, insbesondere linearen bzw. translatorischen, Bewegungsachse bzw. Bewegungsbahn. Andere, d. h. z. B. bogenförmige, Bewegungsachsen bzw. Bewegungsbahnen sind grundsätzlich denkbar.

Sofern mehrere Zuführ- und/oder Abführelemente vorgesehen sind, können diese entlang gleich- oder ungleichförmiger Bewegungsachsen bzw. Bewegungsbahnen bewegt werden. Die Bewegung der Zuführ- und/oder Abführelemente kann zeitgleich oder zeitlich verzögert erfolgen. Zweckmäßig erfolgt die Bewegung mehrerer Zuführ- und/oder Abführelemente jedoch zeitgleich entlang paralleler Bewegungsachsen bzw. Bewegungsbahnen.

Die Erfassungseinrichtung dient der Erfassung einer zumindest abschnittsweisen Änderung wenigstens eines optisch erfassbaren Parameters eines entsprechenden Analysebereichs eines Urinteststreifens. Der optisch erfassbare Parameter ändert sich in Abhängigkeit der Zusammensetzung einer den Analysebereich benetzenden bzw. kontaktierenden Urinmenge in optisch erfassbarer Weise, d. h. z. B. durch Veränderung der Farbe und/oder durch Veränderung der Farbintensität. Sonach handelt es sich bei der Farbe bzw. der Farbintensität des Analysebereichs um einen entsprechenden optisch erfassbaren Parameter. Zur Erfassung einer entsprechenden Änderung des oder eines optisch erfassbaren Parameters umfasst die Erfassungseinrichtung geeignete, insbesondere optische, Erfassungsmittel. Bei einem solchen Erfassungsmittel kann es sich z. B. um einen oder mehrere optische Scanner handeln.

Neben der Erfassung einer zumindest abschnittsweisen Änderung wenigstens eines optisch erfassbaren Parameters eines entsprechenden urinteststreifenseitigen Analysebereichs dient die Erfassungseinrichtung auch der Erzeugung einer Erfassungsinformation. Eine von der Erfassungseinrichtung erzeugte Erfassungsinformation beschreibt wenigstens einen optisch erfassbaren bzw. erfassten Parameter des Analysebereichs oder eine Änderung eines solchen. Mithin kann eine Erfassungsinformation z. B. eine Farbe, eine Farbintensität oder einen Farbumschlag des Analysebereichs beschreiben.

Da die Erfassungseinrichtung zweckmäßig zur Kommunikation mit wenigstens einer Auswertungseinrichtung, welche zur Auswertung einer von der Erfassungseinrichtung erzeugten Erfassungsinformation und zur Ermittlung einer eine Analyse der auf dem Analysebereich des Urinteststreifens befindlichen Urinmenge beschreibenden Auswertungsinformation eingerichtet ist, kann die Erfassungsinformation an entsprechende Auswertungseinrichtungen übermittelt werden, in welchen auf Basis der Erfassungsinformation Rückschlüsse auf die anteilsmäßige bzw. chemische Zusammensetzung der auf den Analysebereich aufgebrachten Urinmenge gezogen werden können. Hierfür umfasst die Vorrichtung zweckmäßig eine der Erfassungseinrichtung zugeordnete Sende- und/oder Empfangseinrichtung, welche eine drahtgebundene oder drahtlose Übermittlung entsprechender Erfassungsinformationen ermöglicht. Die Vorrichtung kann sonach, z. B. mittels Bluetooth, WLAN etc., auch an ein lokales oder globales Datennetzwerk, d. h. z. B. an ein lokales Intranet oder das Internet, angeschlossen bzw. in ein solches eingebunden sein.

Der Betrieb der Zuführ- und Abführeinrichtung wie auch der Betrieb der Erfassungseinrichtung wird typischerweise über eine diesen zugeordnete, vorrichtungsseitige Steuerungseinrichtung gesteuert. In der Steuerungseinrichtung ist typischerweise wenigstens eine Steuerungsvorschrift hinterlegt, gemäß welcher eine konzertierte, d. h. aufeinander abgestimmte, Steuerung des Betriebs der Zuführ- und Abführeinrichtung, der Erfassungseinrichtung sowie gegebenenfalls weiterer, insbesondere der erfindungsgemäßen Vorrichtung zugehöriger, Einrichtungen möglich ist. Zu Letzteren zählen insbesondere die folgenden, im Weiteren noch zu erläuternden Einrichtungen: Druckermittlungseinrichtungen, Pumpeneinrichtungen sowie in eine Bypassleitung geschaltete Ventileinrichtungen.

Über die Steuerungseinrichtung respektive die wenigstens eine in dieser hinterlegte Steuerungsvorschrift lassen sich unterschiedliche Betriebsmodi der erfindungsgemäßen Vorrichtung realisieren. Entsprechende Betriebsmodi sehen insbesondere eine Entnahme einer bestimmten Urinmenge, die Zuführung der oder einer entnommenen bestimmten Urinmenge in eine urinteststreifenseitige Analysekammer, die Abführung der oder einer entnommenen bestimmten Urinmenge aus einer urinteststreifenseitigen Analysekammer, Spülvorgänge der Zuführ- und Abführeinrichtung, Spülvorgänge der Zuführ- und/oder Abführelemente, Spülvorgänge bestimmter in die Zuführ- und/oder Abführelemente führender Leitungsabschnitte bzw. Zuleitungen und Ableitungen, Spülvorgänge eines Urinteststreifens, d. h. insbesondere einer urinteststreifenseitigen Analysekammer, etc. vor.

Entsprechende Betriebsmodi der erfindungsgemäßen Vorrichtung können auf unterschiedliche Weise aktivierbar oder deaktivierbar sein bzw. aktiviert oder deaktiviert werden. Die Aktivierung oder Deaktivierung kann beispielsweise vermittels mobiler Endgeräte, worunter z. B. Handys, Smartphones, Laptops, Tablets etc. zu verstehen sind, erfolgen, welche über eine Kommunikationsverbindung mit einer vorrichtungsseitigen Sende- und/oder Empfangseinrichtung kommunizieren können. Eine Steuerung kann beispielsweise auch per Sprachbefehl erfolgen, wobei die Vorrichtung in diesem Fall mit einer geeigneten Spracherkennungseinrichtung ausgestattet ist. Entsprechende Betriebsmodi der Vorrichtung können selbstverständlich auch voll- oder teilautomatisiert aktiviert oder deaktiviert werden.

Insgesamt ermöglicht die erfindungsgemäße Vorrichtung eine vergleichsweise einfache und integrierte Analyse von Urin und stellt sonach eine Verbesserung gegenüber dem eingangs beschriebenen Stand der Technik dar.

Die erfindungsgemäße Vorrichtung kann, wie im Weiteren noch erläutert wird, einer ein Wasserklosett, kurz ein WC, umfassenden Sanitäreinrichtung zugeordnet respektive in eine solche integriert sein. Mithin können entsprechende Analysen von Urin, wie grundsätzlich auch anderer Körperausscheidungen, in praktischer Weise an der Stelle erfolgen, an welcher Urin ausgeschieden wird. Es ist zur Analyse des Urins sonach nicht mehr nötig, diesen, wie bis dato üblich, in entsprechende Behältnisse umzufüllen, um Urinteststreifen entsprechend zu benetzen. Die Analyse des Urins kann sonach innerhalb der oder einer die erfindungsgemäße Vorrichtung aufnehmenden Sanitäreinrichtung erfolgen.

Zur Realisierung von Bewegungen entsprechender Zuführ- und/oder Abführelemente weist die Zuführ- und Abführeinrichtung zweckmäßig wenigstens eine mit dem wenigstens einen Zuführ- und/oder Abführelement gekoppelte Antriebseinrichtung auf. Über die Antriebseinrichtung ist das wenigstens eine Zuführ- und/oder Abführelement derart gegen den oder einen urinteststreifenseitigen Zu- und/oder Abführbereich bewegbar, dass eine, insbesondere kanülenartige, Spitze des Zuführ- und/oder Abführelements zur Zu- und/oder Abführung der oder einer bestimmten Urinmenge in die oder aus der urinteststreifenseitigen Analysekammer in den urinteststreifenseitigen Zu- und/oder Abführbereich eindringt. Die Antriebseinrichtung umfasst hierfür z. B. einen motorischen Antrieb, d. h. beispielsweise einen Elektromotor, bzw. ist als solcher ausgebildet.

An dieser Stelle ist allgemein zu erwähnen, dass ein entsprechendes Zuführ- und/oder Abführelement typischerweise eine kanülen- oder röhrchenförmige Gestalt aufweist. Ein entsprechendes Zuführ- und/oder Abführelement ist somit typischerweise als ein einen von einem Fluid durchströmbaren Hohlraum umgebender hohlzylindrischer Körper ausgebildet. Die Spitze des Zuführ- und/oder Abführelements kann konstruktiv derart spitz bzw. schräg zulaufend gestaltet sein, dass damit ein Durchdringen bzw. eine Perforation eines einen urinteststreifenseitigen Zuführ- und/oder Abführbereich abdeckenden Elements, insbesondere eines im Weiteren noch zu erläuternden Kapselungselements, möglich ist.

Zweckmäßig umfasst die Vorrichtung wenigstens eine Fördereinrichtung, welche zur Förderung wenigstens eines Urinteststreifens in einen vorrichtungsseitig definierten Erfassungsbereich, in welchem mittels der Erfassungseinrichtung ein Erfassen einer zumindest abschnittsweisen Änderung des wenigstens einen optisch erfassbaren Parameters möglich ist, und/oder aus dem oder einem solchen Erfassungsbereich eingerichtet ist. Über eine solche Fördereinrichtung lässt sich ein oder lassen sich mehrere Urinteststreifen sonach gezielt in einen respektive aus einem entsprechenden Erfassungsbereich der Erfassungseinrichtung befördern. Die Fördereinrichtung ermöglicht sonach eine Einzel- oder Mehrfachförderung entsprechender Urinteststreifen. Die Fördereinrichtung kann zu einer kontinuierlichen oder diskontinuierlichen Förderung entsprechender Urinteststreifen eingerichtet sein.

Zur Förderung entsprechender Urinteststreifen kann die Fördereinrichtung z. B. wenigstens ein drehbar gelagertes Förderelement mit Förderabschnitten zur Förderung wenigstens eines Urinteststreifens umfassen oder als solches ausgebildet sein. Bei dem Förderelement kann es sich z. B. um ein Noppenrad mit durch umfangsmäßig beabstandet angeordnete Noppen ausgebildeten Förderabschnitten zur Förderung wenigstens eines Urinteststreifens handeln. Die typischerweise radial von dem Noppenrad abragenden Noppen definieren sonach Förderabschnitte, mittels welchen Urinteststreifen durch Rotation des Noppenrads befördert werden können. Alternativ oder ergänzend kann die Fördereinrichtung wenigstens ein Förderband mit Förderabschnitten zur Förderung wenigstens eines Urinteststreifens umfassen oder als solches ausgebildet sein. Wiederum alternativ oder ergänzend kann die Fördereinrichtung wenigstens eine Förderrolle, von welcher ein im Weiteren näher erläuterter Verbund mehrerer band- oder gurtförmig miteinander verbundener Urinteststreifen abrollbar oder auf welche ein Verbund mehrerer band- oder gurtförmig miteinander verbundener Urinteststreifen aufrollbar ist, umfassen oder als solche ausgebildet sein. Auch die Förderrollen können jeweils mit Förderabschnitten zur Förderung wenigstens eines Urinteststreifens ausgestattet sein. Insbesondere kann es sich bei den Förderrollen auch um entsprechende Noppenräder handeln.

Die Fördereinrichtung kann insbesondere wenigstens zwei Förderrollen umfassen, so dass ein Verbund mehrerer band- oder gurtförmig miteinander verbundener Urinteststreifen von einer ersten Förderrolle derart abrollbar ist, dass wenigstens ein in den Erfassungsbereich zu fördernder Urinteststreifen in den Erfassungsbereich bewegbar oder bewegt ist, und auf eine zweite Förderrolle derart aufrollbar ist, dass der wenigstens eine Urinteststreifen aus dem Erfassungsbereich bewegbar oder bewegt ist. Es handelt sich hierbei um ein besonders praktisches Prinzip, gemäß welchem unverbrauchte Urinteststreifen in Form eines entsprechenden Verbunds aufgerollt auf einer entsprechenden ersten Förderrolle vorgehalten und von dieser abgerollt werden können. Verbrauchte Urinteststreifen, d. h. insbesondere Urinteststreifen, welche in den Erfassungsbereich bewegt, mittels der Zuführ- und Abführeinrichtung mit Urin kontaktiert und entsprechend mittels der Erfassungseinrichtung "gescannt" wurden, können auf eine zweite Förderrolle aufgerollt und auf dieser gesammelt werden.

Um zu ermitteln, ob ein Urinteststreifen den Erfassungsbereich erreicht hat und sich somit in einer für die Erfassung eines entsprechenden optisch erfassbaren Parameters eines urinteststreifenseitigen Analysebereichs respektive die Erfassung einer Änderung eines solchen erforderlichen Position befindet, kann die Vorrichtung wenigstens eine Positionsermittlungseinrichtung umfassen. Eine solche Positionsermittlungseinrichtung ist entsprechend dazu eingerichtet, eine im Hinblick auf die Erfassung eines entsprechenden optisch erfassbaren Parameters eines urinteststreifenseitigen Analysebereichs respektive die Erfassung einer Änderung eines solchen mittels der Erfassungseinrichtung erforderliche bzw. korrekte Positionierung eines Urinteststreifens zu ermitteln. Die Positionsermittlungseinrichtung kann hierfür z. B. Lichtschranken umfassen oder als solche ausgebildet sein. Eine mittels einer Positionsermittlungseinrichtung realisierte Positionsermittlung eines Urinteststreifens kann sonach im Allgemeinen optisch erfolgen.

Die Vorrichtung kann ferner wenigstens eine der Erfassungseinrichtung nachgeschaltete Abtrenneinrichtung, welche zur Abtrennung wenigstens eines Urinteststreifens aus einem Verbund mehrerer band- oder gurtförmig miteinander verbundener Urinteststreifen eingerichtet ist, umfassen. Mithin können einzelne oder mehrere entsprechend verbrauchte Urinteststreifen mittels einer solchen Abtrenneinrichtung aus dem Verbund gelöst und somit gesondert gehandhabt, d. h. insbesondere entsorgt, werden. Die Abtrenneinrichtung dient also im Allgemeinen dazu, verbrauchte Urinteststreifen von unverbrauchten Urinteststreifen zu trennen und aus einem entsprechenden Verbund zu lösen. Die Abtrenneinrichtung kann hierfür z. B. eine Schneide oder einen Laser umfassen oder als ein(e) solche(r) ausgebildet sein.

Die Vorrichtung umfasst zweckmäßig wenigstens ein Gehäuseteil, welches zur Aufnahme der Zu- und Abführeinrichtung, der Erfassungseinrichtung sowie gegebenenfalls weiterer, d. h. auch im Folgenden noch zu nennender, Einrichtungen der Vorrichtung ausgebildet ist. Das Gehäuseteil ermöglicht sonach eine vergleichsweise kompakte Aufnahme der der Vorrichtung zugehörigen Einrichtungen.

An dem Gehäuseteil kann wenigstens ein Aufnahmeabteil, welches zur Aufnahme von verbrauchten, d. h. insbesondere zwischenzeitlich mit einer bestimmten Urinmenge befüllten Urinteststreifen, insbesondere nach der Erfassung einer zumindest abschnittsweisen Änderung des wenigstens einen optisch erfassbaren Parameters des Analysebereichs des Urinteststreifens mittels der Erfassungseinrichtung, ausgebildet ist, lösbar befestigbar oder befestigt sein.

An dem Gehäuseteil kann ferner wenigstens ein Lagerabschnitt zur Lagerung wenigstens einer entsprechenden Fördereinrichtung, insbesondere in Form einer Förderrolle, ausgebildet sein. Bei dem Lagerabschnitt kann es sich konstruktiv um einen bolzen- oder stiftartigen Vorsprung handeln, auf welchem eine entsprechende Fördereinrichtung, insbesondere in Form einer Förderrolle, drehbar lagerbar oder gelagert ist. Der bolzen- oder stiftartige Vorsprung bildet dabei typischerweise die Drehachse der Fördereinrichtung, d. h. insbesondere der Förderrolle.

Insbesondere im Zusammenhang mit der weiter oben erwähnten Spülung der Zuführ- und Abführeinrichtung sowie im Allgemeinen verschiedener Betriebsmodi der Vorrichtung respektive einer diese umfassenden Sanitäreinrichtung kann es zweckmäßig sein, wenn wenigstens eine Bypassleitung vorhanden ist, welche eine in ein Zuführelement führende Zuleitung und eine aus einem Abführelement führende Ableitung verbindet. In die Bypassleitung ist zweckmäßig wenigstens eine Ventileinrichtung zum Öffnen und Schließen der Bypassleitung geschaltet.

Um Urin durch die Vorrichtung zu befördern, d. h. insbesondere in eine entsprechende urinteststreifenseitige Analysekammer zuzuführen bzw. aus einer entsprechenden urinteststreifenseitigen Analysekammer abzuführen, ist zweckmäßig wenigstens eine in eine in ein Zuführelement führende Zuleitung geschaltete Pumpeneinrichtung und/oder wenigstens eine in eine aus einem Abführelement führende Ableitung geschaltete Pumpeneinrichtung vorhanden. Die wenigstens eine in eine in ein Zuführelement führende Zuleitung geschaltete Pumpeneinrichtung ist zur Förderung einer bestimmten Urinmenge in den oder einen urinteststreifenseitigen Zuführbereich bzw. in eine entsprechende urintestreifenseitige Analysekammer eingerichtet. Die wenigstens eine in eine aus einem Abführelement führende Ableitung geschaltete Pumpeneinrichtung ist zur Förderung einer bestimmten Urinmenge aus dem oder einem urinteststreifenseitigen Abführbereich bzw. aus einer entsprechenden urintestreifenseitige Analysekammer eingerichtet. Die Pumpeneinrichtungen können übliche Pumpen, wie z. B. Drehkreisel- Kreiskolbenpumpen etc., umfassen oder als solche ausgebildet sein.

Die Vorrichtung kann ferner wenigstens eine Druckermittlungseinrichtung, welche zur Ermittlung des Druckes der oder einer in den urinteststreifenseitigen Zuführbereich geführten Urinmenge, insbesondere des Druckes der durch das oder ein Zuführelement geführten Urinmenge eingerichtet ist, umfassen. Anhand des über eine solche Druckermittlungseinrichtung ermittelbaren Drucks einer in den urinteststreifenseitigen Zuführbereich geführten Urinmenge lassen sich Rückschlüsse auf etwaig vorhandene Gasblasen, insbesondere Luftblasen, innerhalb der Urinmenge ziehen, welche die Aussagekraft der Erfassungsinformation gegebenenfalls beinträchtigen können.

Beschrieben wird ferner eine Urinteststreifeneinrichtung, insbesondere für eine wir vorstehend beschriebene Vorrichtung, umfassend wenigstens einen Urinteststreifen mit einer wenigstens einen Analysebereich zur Analyse von Urin aufweisenden Analysekammer. Dabei ist der urinteststreifenseitige Analysebereich unter Ausbildung einer zumindest abschnittsweise fluiddichten Kapselung zumindest abschnittsweise von wenigstens einem Kapselungselement umgeben.

Der grundsätzliche Aufbau der Urinteststreifeneinrichtung umfasst wenigstens einen Urinteststreifen. Der Urinteststreifen umfasst wenigstens eine Analysekammer, welche wenigstens einen Analysebereich zur Analyse von Urin aufweist. Der Analysebereich umfasst wenigstens ein Analysereagenz bzw. ist durch wenigstens ein solches gebildet.

Bei dem Analysereagenz handelt es sich im Allgemeinen um eine chemisch reaktive Substanz, welche bei Kontakt mit Urin eine chemische Reaktion derart vollzieht, dass eine optisch erfassbare Änderung wenigstens eines optisch erfassbaren Parameters erfolgt. Der optisch erfassbare Parameter der chemisch reaktiven Substanz bzw. des Analysereagenz ändert sich also in Abhängigkeit der Zusammensetzung einer diese(s) kontaktierenden Urinmenge in optisch erfassbarer Weise, d. h. z. B. durch Veränderung der Farbe und/oder durch Veränderung der Farbintensität. Sonach handelt es sich bei der Farbe, der Farbintensität oder einem Farbumschlag des Analysebereichs um einen optisch erfassbaren Parameter des Analysebereichs.

Die Urinteststreifeneinrichtung zeichnet sich insbesondere durch eine wenigstens abschnittsweise, insbesondere vollständige, fluiddichte Kapselung des Analysebereichs aus. Die Kapselung ist durch wenigstens ein Kapselungselement realisiert, welches den Analysebereich oder bestimmte Abschnitte des Analysebereichs umgibt. Je nach Anordnung und geometrischer Gestaltung des Kapselungselements kann zwischen diesem und dem Analysebereich ein bestimmter räumlicher Abstand ausgebildet sein, welcher die oder einen Teil der urinteststreifenseitigen Analysekammer bildet. Entsprechend kann das Kapselungselement wenigstens einen Teil der urinteststreifenseitigen Analysekammer begrenzen.

In allen Fällen ist der Analysebereich, welcher gegebenenfalls auf einem Trägerelement aufgebracht bzw. ausgebildet ist, sonach mittels des oder eines Kapselungselements gegenüber äußeren Einflüssen, welche die Qualität eines entsprechenden Analysereagenz, welches bei Kontakt mit Urin, eine optisch erfassbare Änderung eines optisch erfassbaren Parameters, d. h. z. B. einen Farbumschlag, erfährt, beeinträchtigen können, geschützt. Entsprechende äußere Einflüsse sind insbesondere durch Feuchtigkeit und Nässe gegeben, da diese zu einer Degradation entsprechender Analysebereiche respektive entsprechenden diese bildenden Analysereagenzien führen können. Derart begegnet die Urinteststreifeneinrichtung auch der bisher bestehenden Problematik einer langfristigen Lagerung von Urinteststreifen, bei welcher sich eine Degradation der Analysebereiche bisher nur schwer ausschließen ließ. Entsprechende äußere Einflüsse können auch durch mechanische Einwirkungen gegeben sein, welche ohne entsprechende Kapselung zu Beschädigungen des Analysebereichs führen können.

Unter einem Kapselungselement kann auch eine mehrteilige, insbesondere mehrlagige oder mehrschichtige, Struktur zu verstehen sein. Dies kann insbesondere dann zweckmäßig sein, wenn eine fluiddichte Kapselung gegenüber chemisch unterschiedlichen Fluiden realisiert werden soll, so dass bestimmte Lagen oder Schichten des Kapselungselements eine gezielte Kapselung gegenüber bestimmten Fluiden ermöglicht.

Der Analysebereich ist zweckmäßig zwischen einem Zuführbereich zum Zuführen einer bestimmten Fluidmenge, insbesondere Urinmenge, in die Analysekammer und einem Abführbereich zum Abführen einer bestimmten Fluidmenge, insbesondere Urinmenge, aus der Analysekammer ausgebildet. Zweckmäßig ist der Zuführbereich und/oder der Abführbereich unter Ausbildung einer zumindest abschnittsweise fluiddichten Kapselung zumindest abschnittsweise von dem oder wenigstens einem weiteren Kapselungselement umgeben. Mithin ist nicht nur eine Kapselung des Analysebereichs, sondern auch eine Kapselung entsprechender mit dem Analysebereich kommunizierender bzw. verbundener Zuführ- und/oder Abführbereiche realisierbar.

Um ein Einführen eines Zuführ- und/oder Abführelements in entsprechende urinteststreifenseitige Zuführ- und/oder Abführbereiche zu erleichtern, ist es zweckmäßig, wenn der Zuführbereich und/oder der Abführbereich eine, insbesondere kalotten- bzw. kugelsegmentförmige, Ausbuchtung aufweist. Derart kann ebenso ein Durchströmen der Analysekammer erleichtert sein, was die Analyse und somit die Aussagekraft des Urinteststreifens verbessern kann. Entsprechende, insbesondere kalotten- bzw. kugelsegmentförmige, Ausbuchtungen können auch als so genanntes Einstichauge bezeichnet bzw. erachtet werden. Eine entsprechende Ausbuchtung kann alternativ zu kalotten- bzw. kugelsegmentförmigen Geometrien beispielsweise auch konische, polyedrische oder zylindrische Geometrien aufweisen.

Das Kapselungselement kann in wenigstens zwei Kapselungselementabschnitte unterteilt sein. Die wenigstens zwei Kapselungselementabschnitte bilden das Kapselungselement. Dabei kann das Kapselungselement einen die freiliegende Fläche des Analysebereichs umgebenden oberen Kapselungselementabschnitt und einen die der freiliegenden Oberfläche des Analysebereichs abgewandte Fläche des Analysebereichs umgebenden unteren Kapselungselementabschnitt aufweisen.

Die oberen und unteren Kapselungselementabschnitte können sich z. B. in ihrer geometrisch-konstruktiven Abmessung, ihrer Form und ihrer Materialität unterscheiden. Insbesondere kann es sich bei einem entsprechenden oberen Kapselungselementabschnitt um ein dreidimensional komplex geformtes Formteil handeln, während es sich bei einem entsprechenden unteren Kapselungselementabschnitt um ein flächiges, sich im Wesentlichen zweidimensional erstreckendes Flächenteil, d. h. z. B. um eine Folie, handeln kann.

Der obere Kapselungselementabschnitt ist typischerweise zumindest abschnittsweise aus einem transparenten Material, insbesondere einem transparenten Kunststoffmaterial, ausgebildet. Dies ist insofern zweckmäßig als derart eine Erfassung einer Änderung eines optisch erfassbaren Parameters, d. h. z. B. ein Farbumschlag, des Analysebereichs möglich ist. Mithin ist der Begriff "transparent" insbesondere derart zu verstehen, dass der obere Kapselungselementabschnitt eine Erfassung eines optisch erfassbaren Parameters zulässt. Als geeignete Materialien zur Ausbildung des oberen Kapselungselementabschnitts kommen insbesondere transparente Kunststoffe, wie z. B. PC, PMMA, in Betracht.

Der untere Kapselungselementabschnitt muss dagegen nicht zwingend aus einem transparenten Material ausgebildet sein. Zweckmäßig ist es hier, wenn der untere Kapselungselementabschnitt zumindest in den dem Zuführbereich und/oder dem Abführbereich gegenüber liegenden Bereichen derart elastisch ausgebildet ist, dass er ein diesen perforierendes, in den Zuführ- und/oder Abführbereich eindringendes Zuführ- und/oder Abführelement abschnittsweise dichtend umschließt. Der untere Kapselungselementabschnitt kann sich also an ein in den Zuführ- bzw. Abführbereich eindringendes Zuführ- bzw. Abführelement anschmiegen. Derart kann sichergestellt werden, dass während des Zuführens bzw. Abführens einer bestimmten Urinmenge in den bzw. aus dem Urinteststreifen keine Leckagen auftreten.

Als zur Ausbildung des unteren Kapselungselementabschnitts geeignete, d. h. entsprechend elastische, Materialien kommen insbesondere Kunststoffe, wie z. B. PE und/oder PET, in Frage. Denkbar ist auch die Verwendung thermoplastischer Elastomere, wie z. B. TPO und/oder TPV, welche sich strukturbedingt durch eine vergleichsweise hohe Elastizität und somit hochelastische Eigenschaften auszeichnen. Die Materialien liegen typischerweise als Folien mit einer Stärke bzw. Dicke in einem Bereich zwischen 30 und 120 µm, insbesondere in einem Bereich zwischen 70 und 110µm, vor.

Die elastischen Eigenschaften entsprechender den unteren Kapselungselementabschnitt bildender Folien, d. h. insbesondere Kunststofffolien, können im Rahmen deren Herstellung bzw. Verarbeitung, z. B. durch Strecken, gezielt beeinflusst werden. Zur Ausbildung des unteren Kapselungselementabschnitts ist prinzipiell auch die Verwendung von metallischen Folien oder Verbundfolien, welche aus aus unterschiedlichen Materialien gebildeten Verbundmaterialien, z. B. Kunststoff und Metall, gebildet sind, denkbar.

Gleichermaßen ist es zweckmäßig, wenn der untere Kapselungselementabschnitt zumindest in den dem Zuführbereich und/oder dem Abführbereich gegenüber liegenden Bereichen derart elastisch ausgebildet ist, dass ein perforierter Bereich durch die diesen begrenzenden Materialabschnitte des unteren Kapselungselementabschnitts dichtend verschlossen ist. Der untere Kapselungselementabschnitt kann sonach zumindest bereichsweise als Septum bzw. als Verschlussmembran ausgebildet sein. Zur Ausbildung des unteren Kapselungselementabschnitts kommen insbesondere die vorstehend genannten Materialien, d. h. insbesondere Kunststoffmaterialien bzw. Verbundmaterialien, in Betracht.

Zweckmäßig umfasst die Urinteststreifeneinrichtung nicht nur einen, sondern mehrere Urinteststreifen. Die Urinteststreifen sind dabei unter Ausbildung eines mehrere Urinteststreifen umfassenden Verbunds band- oder gurtförmig miteinander verbunden. Ein entsprechender Verbund beinhaltet sonach eine Vielzahl an Urinteststreifen. Dies bringt einerseits den Vorteil mit sich, dass die erfindungsgemäße Vorrichtung lediglich in vergleichsweise großen Zeitabständen mit Urinteststreifen bestückt werden muss. Die Sicherung des Betriebs der Vorrichtung kann derart über einen vergleichsweise langen Zeitraum sichergestellt werden. Andererseits ergeben sich aus der einfachen Handhabung eines entsprechenden Verbunds, welcher z. B. auf einfache Weise auf eine Fördereinrichtung in Form einer Förderrolle aufgerollt bzw. von einer solchen abgerollt werden kann, praktische Vorteile.

Die Verbindung respektive der Verbund zwischen entsprechenden Urinteststreifen kann dabei insbesondere über jeweilige Kapselungselemente gebildet sein, welche unter Ausbildung kontinuierlicher oder diskontinuierlicher Verbindungsbereiche zumindest abschnittsweise miteinander verbunden sein können. Die Kapselungselemente können in den Verbindungsbereichen grundsätzlich form- und/oder kraft- und/oder stoffschlüssig miteinander verbunden sein. Konkret können die Kapselungselemente jeweiliger Urinteststreifen z. B. miteinander verklebt oder verschweißt sein.

Die Verbindung der Urinteststreifen kann für den beispielhaften Fall, in dem entsprechende Urinteststreifen jeweils eine rechteckige Grundform aufweisen, über jeweilige Längsseiten der Urinteststreifen erfolgen. Selbstverständlich kann der Verbund bei entsprechenden Urinteststreifen mit rechteckigen Grundformen auch über jeweilige Stirnseiten erfolgen. Der Ort der Verbindung entsprechender Urinteststreifen bestimmt sich grundsätzlich nach deren jeweiliger Grundform, in Abhängigkeit welcher geeignete Verbindungsbereiche festzulegen sind. Dabei ist insbesondere auch die Art der Lagerung und Zuführung eines Verbunds entsprechender Urinteststreifen in eine vorrichtungsseitige Erfassungseinrichtung zu beachten.

Die Erfindung betrifft ferner eine Sanitäreinrichtung, umfassend ein, insbesondere wandhängendes oder bodenstehendes, WC mit einem, insbesondere keramischen, Grundkörper und mit einer wie weiter oben beschriebenen Vorrichtung. Im Allgemeinen gelten hinsichtlich der Sanitäreinrichtung demzufolge sämtliche Ausführungen hinsichtlich der Vorrichtung analog.

Die erfindungsgemäße Sanitäreinrichtung umfasst ein WC in wandhängender oder bodenstehender Ausführung. Das WC ist sonach an einer Wand oder einem Boden befestigbar bzw. montierbar. Das WC umfasst einen, insbesondere keramischen, Grundkörper. Bezogen auf den ordnungsgemäß montierten Zustand des Grundkörpers weist dieser insbesondere obere Flächenabschnitte zur Montage eines Deckel- und/oder Sitzteils sowie innere Flächenabschnitte, welche einen inneren Bereich, in welchen von einem Benutzer Ausscheidungen, d. h. insbesondere Fäkalien und Urin, abgegeben werden können auf.

Im Hinblick auf die Vorrichtung umfasst das WC Aufnahme- oder Befestigungsbereiche zur Aufnahme oder Befestigung der Vorrichtung oder einzelner Bestandteile der Vorrichtung. Grundsätzlich ist die Vorrichtung, d. h. insbesondere ein der Vorrichtung gegebenenfalls zugehöriges Gehäuseteil, form- und/oder kraft- und/oder stoffschlüssig an dem Grundkörper befestigbar oder befestigt ist. Hierfür sind in entsprechenden WC-seitigen Aufnahme- oder Befestigungsbereichen geeignete Befestigungsmittel, welche z. B. eine Rast-, Schraub- oder Klebeverbindung der Vorrichtung an dem WC-seitigen Grundkörper ermöglichen, vorhanden. Selbstverständlich können entsprechende Befestigungsmittel auch vorrichtungsseitig vorhanden sein.

Im Hinblick auf eine konkrete geometrisch-konstruktive Gestaltung eines Grundkörpers sind entsprechende WC-seitige Aufnahme- bzw. Befestigungsbereiche zweckmäßig so anzuordnen, dass die Vorrichtung für einen Benutzer nicht sichtbar ist. Hierfür können Abdeckelemente, z. B. in Form von Blenden, vorgesehen werden, die ein Abdecken der Vorrichtung ermöglichen. Entsprechende Abdeckelemente können durch geeignete Befestigungselemente, z. B. in Form von Befestigungswinkeln, in ihrer Lage und Position relativ zu dem WC-seitigen Grundkörper fixiert werden.

Um eine Entnahme einer bestimmten Urinmenge, welche mittels der Vorrichtung analysiert werden soll, zu ermöglichen, ist in dem Grundkörper wenigstens eine Entnahmeeinrichtung ausgebildet oder angeordnet. Die Entnahmeeinrichtung umfasst wenigstens ein in einer Öffnung des Grundkörpers angeordnetes Rohrelement, welches zwischen einer Öffnungs- und einer Schließstellung bewegbar relativ zu einem Schließelement gelagert ist. Das Schließelement ist typischerweise lagefest an dem Grundkörper angeordnet bzw. befestigt. In der Öffnungsstellung ist ein Durchströmen des Rohrelements möglich. Die Schließstellung definiert sich sonach dadurch, dass in dieser ein Durchströmen des Rohrelements nicht möglich ist. Über eine konzertierte Steuerung der Bewegung des Rohrelements gegen das Schließelement, welche z. B. durch ein Bewegen des Rohrelements in die Öffnungsstellung und ein nach Ablaufen eines bestimmten Zeitintervalls, z. B. ein Zeitintervall zwischen 1 und 5 Sekunden, erfolgendes Bewegen des Rohrelements in die Schließstellung realisiert sein kann, lässt sich sonach eine bestimmte Urinmenge entnehmen.

Bewegungen des Rohrelements werden zweckmäßig über eine mit diesem gekoppelte Antriebseinrichtung realisiert. Das Rohrelement ist über die Antriebseinrichtung insbesondere zwischen der Öffnungs- und der Schließstellung bewegbar. Die Antriebseinrichtung umfasst hierfür z. B. einen magnetischen Antrieb, d. h. beispielsweise einen Drehmagneten, oder einen motorischen Antrieb, d. h. beispielsweise einen Elektromotor, bzw. ist als solcher ausgebildet. Zur Bewegung des Rohrelements in die Schließstellung respektive zur Unterstützung der Bewegung des Rohrelements in die Schließstellung können ferner Federmittel vorgesehen sein, über welche das Rohrelement mit einer dieses in die Schließstellung bewegenden oder in der Schließstellung sichernden Federkraft beaufschlagbar ist.

Selbstverständlich ist prinzipiell auch eine umgekehrte Kinematik, gemäß welcher ein bewegbar gelagertes Schließelement zwischen einer Öffnungs- und einer Schließstellung relativ zu einem lagefest an dem Grundkörper angeordneten bzw. befestigten Rohrelement bewegbar ist, vorstellbar.

Die Entnahmeeinrichtung, d. h. insbesondere das dieser zugehörige Schließelement, ist typischerweise derart angeordnet, dass sie zumindest abschnittsweise in entsprechende innere Flächenabschnitte des Grundkörpers ragt. In der Schließstellung schließt die Entnahmeeinrichtung, d. h. insbesondere das dieser zugehörige Schließelement, typischerweise bündig mit inneren Flächenabschnitten des Grundkörpers ab, so dass sich (im Wesentlichen) eine einheitliche Oberfläche ergibt.

Das Rohrelement ist typischerweise über eine Leitungsverbindung mit einer in die vorrichtungsseitige Zuführ- und Abführeinrichtung führenden Zuleitung verbunden, über welche eine mittels der Entnahmeeinrichtung entnommene Urinmenge in die Zuführ- und Abführeinrichtung und weiter in eine urinteststreifenseitige Analysekammer geführt werden kann. Entsprechend weist das Rohrelement an oder im Bereich seines dem Grundkörper abgewandten freien Endes zweckmäßig ein Anschlussmittel zum Anschluss an die oder eine vorrichtungsseitige Zuführ- und Abführeinrichtung auf.

In eine in eine entsprechende vorrichtungsseitige Zuführ- und Abführeinrichtung führende Zuleitung kann wenigstens ein Tank geschaltet sein, in dem eine bestimmte Urinmenge, z. B. ca. 200 ml, vor der eigentlichen Zuführung in die vorrichtungsseitige Zuführ- und Abführeinrichtung gesammelt und vorgehalten werden kann. Der oder ein entsprechender Tank ist sonach zwischen die Entnahmeeinrichtung und die vorrichtungsseitige Zuführ- und Abführeinrichtung geschaltet. Der oder ein entsprechender Tank kann mit wenigstens einer Belüftungs- und/oder wenigstens einer Füllstandsermittlungseinrichtung versehen sein. Eine Belüftungseinrichtung kann z. B. ein Belüftungsventil umfassen oder als solches ausgebildet sein. Eine Füllstandsermittlungseinrichtung kann z. B. einen Füllstandssensor umfassen oder als solcher ausgebildet sein.

Insbesondere nach der Analyse des Urins ist es sinnvoll, dieses wieder in das WC zurückzuführen und so einem WC-seitigen Abfluss zuzuführen. Entsprechend ist es zweckmäßig, wenn eine aus der oder einer vorrichtungsseitigen Abführeinrichtung führende Ableitung in einen durch den Grundkörper begrenzten Innenraum führt. Der grundkörperseitige Innenraum umfasst entsprechende innere Flächenabschnitte des Grundkörpers.

Die Sanitäreinrichtung respektive das dieser zugehörige WC umfasst typischerweise wenigstens eine Spüleinrichtung zur Durchführung eines Spülvorgangs des WCs. Dabei ist zweckmäßig wenigstens eine spüleinrichtungsseitige Fluidleitung mit der oder einer in eine vorrichtungsseitige Zuführeinrichtung führenden Zuleitung verbindbar oder verbunden. Derart lassen sich Spül- bzw. Reinigungsvorgänge der Vorrichtung realisieren, was z. B. sinnvoll ist, um eine unerwünschte Ausbildung von Ablagerungen und/oder Gerüchen zu verhindern.

Neben der Spüleinrichtung kann die Sanitäreinrichtung respektive das dieser zugehörige WC auch eine Duscheinrichtung umfassen, mittels welcher nach Benutzen der Sanitäreinrichtung eine Reinigung bestimmter benutzerseitiger Körperbereiche vorgenommen werden können. Entsprechend kann auch eine Trocknungseinrichtung vorgesehen sein, welche eine Trocknung entsprechend mittels der Duscheinrichtung gereinigter benutzerseitiger Körperbereiche ermöglicht.

Weitere Vorteile, Merkmale und Einzelheiten der Erfindung ergeben sich aus den im Folgenden beschriebenen Ausführungsbeispielen sowie anhand der Zeichnungen. Dabei zeigen:
- Fig. 1 - 5: jeweils eine Prinzipdarstellung einer Sanitäreinrichtung gemäß einem Ausführungsbeispiel der Erfindung;
- Fig. 6: eine Prinzipdarstellung einer Entnahmeeinrichtung einer Sanitäreinrichtung gemäß einem Ausführungsbeispiel der Erfindung;
- Fig. 7 - 10: jeweils eine Prinzipdarstellung einer Vorrichtung zur Analyse von Urin gemäß einem Ausführungsbeispiel der Erfindung;
- Fig. 11 - 13: jeweils eine Prinzipdarstellung einer Urinteststreifeneinrichtung; und
- Fig. 14 - 19: jeweils eine schematische Darstellung unterschiedlicher Betriebsmodi einer Sanitäreinrichtung gemäß einem Ausführungsbeispiel der Erfindung.

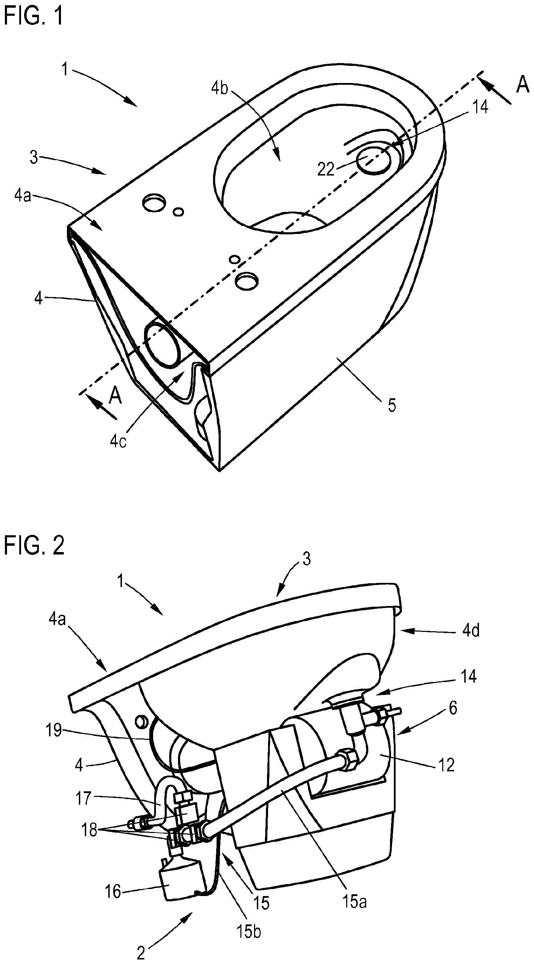

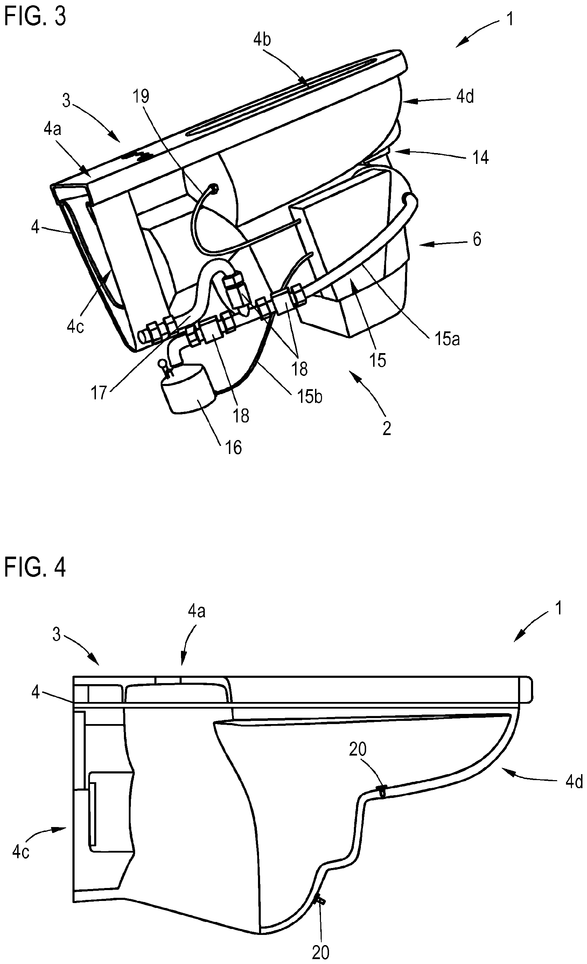

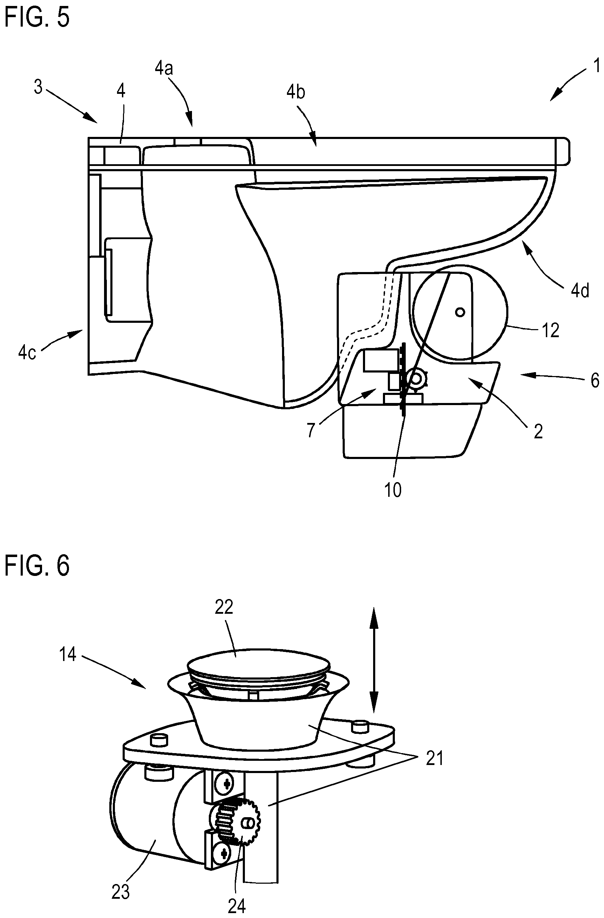

Die Fig. 1 - 5 zeigen jeweils eine Prinzipdarstellung einer Sanitäreinrichtung 1 gemäß einem Ausführungsbeispiel der Erfindung. Ersichtlich zeigen die Fig. 1 - 3 dabei jeweils perspektivische Ansichten und die Fig. 4, 5 jeweils Schnittansichten der Sanitäreinrichtung 1. Die Schnittrichtung sowie die Blickrichtung sind in Fig. 1 durch die allgemeine Schnittlinie A - A angedeutet. Die Sanitäreinrichtung 1 ist mit einer Vorrichtung 2 zur Analyse von Urin ausgestattet. Über die Integration einer solchen Vorrichtung 2 in die Sanitäreinrichtung 1 lässt sich auf einfache Weise eine Analyse bzw. eine Untersuchung von in die Sanitäreinrichtung 1 abgegebenem Urin oder anderen Körperflüssigkeiten vornehmen.

Die Sanitäreinrichtung 1 umfasst ein bodenstehendes oder wandhängendes WC 3. Das WC 3 umfasst einen keramischen Grundkörper 4. Der Grundkörper 4 weist verschiedene Flächenabschnitte auf, von welchen im Weiteren lediglich die Flächenabschnitte 4a - 4d näher beschrieben werden. Bezogen auf den ordnungsgemäß montierten Zustand des Grundkörpers 4 weist dieser obere Flächenabschnitte 4a zur Montage eines Deckel- und/oder Sitzteils (nicht gezeigt), innere Flächenabschnitte 4b, welche einen inneren schüsselartigen Bereich, in welchen von einem Benutzer Ausscheidungen bzw. Körperflüssigkeiten, d. h. insbesondere Fäkalien und Urin, abgegeben werden können, begrenzen, hintere Flächenabschnitte 4c, über welche ein Anschluss an eine mit einer Kanalisation verbundene Rohrleitung (nicht gezeigt) möglich ist, und vordere Flächenabschnitte 4d auf.

Die Vorrichtung 2 ist in Fig. 1 nicht sichtbar, da sie sich hinter einem Abdeckelement 5 in Form einer keramischen Blende befindet. Das Abdeckelement 5 kann durch geeignete Befestigungselemente (nicht gezeigt), z. B. in Form von Befestigungswinkeln, in seiner Lage und Position relativ zu dem WC-seitigen Grundkörper 4 fixiert werden. In den Fig. 2, 3 ist das Abdeckelement 5 entfernt, so dass sich die Vorrichtung 2 erkennen lässt respektive sich dieser zugehörige Bestandteile erkennen lassen.

Die Fig. 4 - 5 zeigen die Vorrichtung 2 respektive ein dieser zugehöriges Gehäuseteil 6, in oder an welchem die wesentlichen Bestandteile bzw. Funktionskomponenten der Vorrichtung 2 aufgenommen bzw. gelagert sind ohne ein entsprechendes Abdeckelement 5.

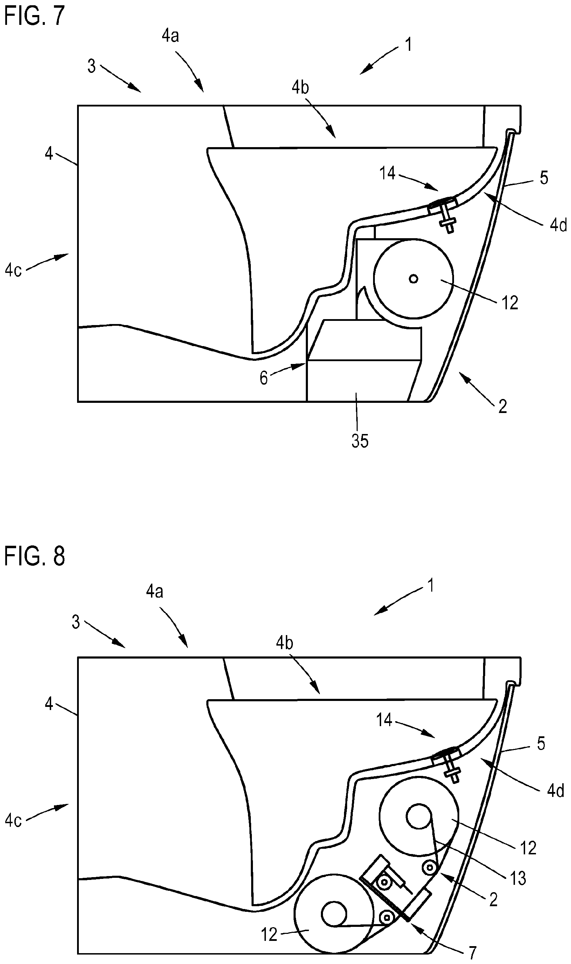

Zu den wesentlichen Bestandteilen bzw. Funktionskomponenten der Vorrichtung 2 gehört eine Zuführ- und Abführeinrichtung 7, welche zur Zuführung einer bestimmten Urinmenge in eine wenigstens einen Analysebereich 8 aufweisende Analysekammer 9 eines Urinteststreifens 10 und zur Abführung einer bestimmten Urinmenge aus einer bzw. der wenigstens einen Analysebereich 8 aufweisenden Analysekammer 9 eines bzw. des Urinteststreifens 10 eingerichtet ist. Aufbau und Funktion der Zuführ- und Abführeinrichtung 7 werden insbesondere im Zusammenhang mit den Fig. 7 - 10 näher beschrieben.

Zu den wesentlichen Bestandteilen bzw. Funktionskomponenten der Vorrichtung 2 gehört ferner eine Erfassungseinrichtung 11, welche zur Erfassung einer zumindest abschnittsweisen Änderung wenigstens eines optisch erfassbaren Parameters des oder eines entsprechenden Analysebereichs 8 des oder eines Urinteststreifens 10 eingerichtet ist. Der optisch erfassbare Parameter ändert sich in Abhängigkeit der Zusammensetzung einer einen entsprechenden urinteststreifenseitigen Analysebereich 8 kontaktierenden Urinmenge in optisch erfassbarer Weise, d. h. z. B. durch Veränderung der Farbe und/oder durch Veränderung der Farbintensität. Sonach handelt es sich z. B. bei der Farbe bzw. der Farbintensität des Analysebereichs 8 um einen entsprechenden optisch erfassbaren Parameter. Die Erfassungseinrichtung 11 ist ferner zur Erzeugung einer Erfassungsinformation, welche wenigstens einen solchen optisch erfassbaren bzw. erfassten Parameter eines Analysebereichs 8 oder eine Änderung eines solchen beschreibt, eingerichtet. Die Erfassungseinrichtung 11 umfasst zur Erfassung einer entsprechenden Änderung eines optisch erfassbaren Parameters optische, Erfassungsmittel in Form von optischen Scannern. Die Funktion der Erfassungseinrichtung 11 wird ebenso insbesondere im Zusammenhang mit den Fig. 7 - 10 näher beschrieben.

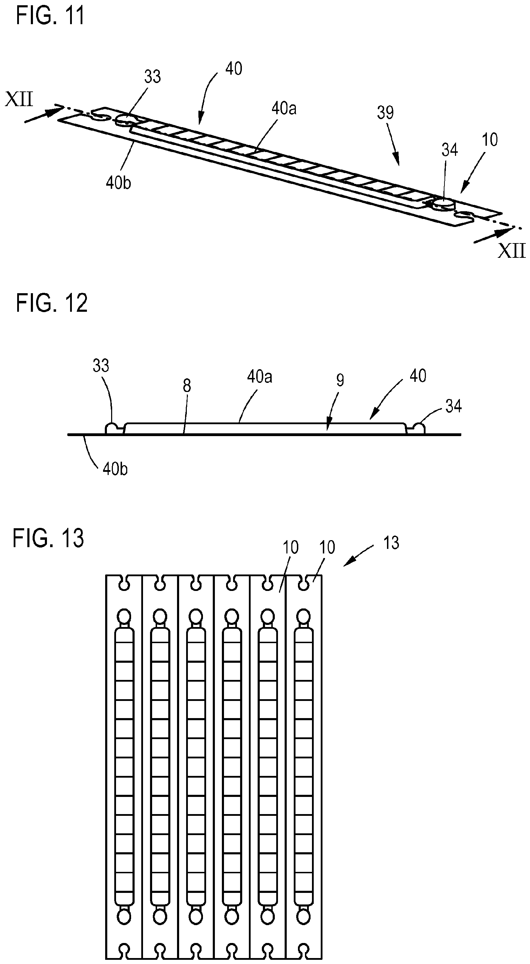

Wie insbesondere anhand der Fig. 2 und 5 ersichtlich ist, ist an dem Gehäuseteil 6 der Vorrichtung 2 eine Fördereinrichtung 12 in Form einer Förderrolle drehbar gelagert. Die Fördereinrichtung 12 ist zur Förderung wenigstens eines Urinteststreifens 10 in einen vorrichtungsseitig definierten Erfassungsbereich, in welchem mittels der Erfassungseinrichtung 11 ein Erfassen einer zumindest abschnittsweisen Änderung eines entsprechenden optisch erfassbaren Parameters eines urinteststreifenseitigen Analysebereichs 8 möglich ist, und/oder aus einem solchen Erfassungsbereich eingerichtet. Von der als Förderrolle ausgebildeten Fördereinrichtung 12 bzw. auf die als Förderrolle ausgebildete Fördereinrichtung 12 ist ein insbesondere im Zusammenhang mit Fig. 13 näher beschriebener Verbund 13 mehrerer band- oder gurtförmig miteinander verbundener Urinteststreifen 10 abrollbar bzw. aufrollbar.

Fig. 1 zeigt ferner eine WC-grundkörperseitige Entnahmeeinrichtung 14. Die Entnahmeeinrichtung 14 ist innerhalb einer Öffnung im Bereich der inneren Flächenabschnitte 4b des Grundkörpers 4 angeordnet. Die Entnahmeeinrichtung 14 dient im Rahmen des Urinierens eines Benutzers in das WC 3 der Entnahme einer bestimmten Urinmenge, welche der Vorrichtung 2 zu Analysezwecken zugeführt werden soll. Aufbau und Funktion der Entnahmeeinrichtung 14 wird mit Bezug auf Fig. 6 näher beschrieben.

Anhand der Fig. 2, 3 ist ersichtlich, dass von der Entnahmeeinrichtung 14 ein erster Leitungsabschnitt 15a einer Zuleitung 15 in die vorrichtungsseitige Zuführ- und Abführeinrichtung 7 zu einem Tank 16 führt. Es ist also in eine in die Vorrichtung 2 führende Zuleitung 15 ein Tank 16 geschaltet, in dem eine bestimmte Urinmenge, z. B. ca. 200 ml, vor der eigentlichen Zuführung in die Vorrichtung 2 respektive in die vorrichtungsseitige Zuführ- und Abführeinrichtung 7 gesammelt und vorgehalten werden kann. Der Tank 16 ist sonach zwischen die Entnahmeeinrichtung 14 und die vorrichtungsseitige Zuführ- und Abführeinrichtung 7 geschaltet. Der Tank 16 ist mit einer nicht näher bezeichneten Belüftungseinrichtung in Form eines Belüftungsventils und einer nicht näher bezeichnete Füllstandsermittlungseinrichtung in Form eines Füllstandssensors ausgestattet.

Ausgehend von dem Tank 16 führt ein zweiter Leitungsabschnitt 15b der Zuleitung 15 in die vorrichtungsseitige Zuführ- und Abführeinrichtung 7. Bei dem ersten Leitungsabschnitt 15a der Zuleitung 15 handelt es sich z. B. um einen flexiblen Gewebeschlauch, bei dem zweiten Leitungsabschnitt 15b der Zuleitung 15 handelt es sich z. B. um einen Laborschlauch mit einem im Vergleich zu dem oder einem Gewebeschlauch geringeren Durchmesser.

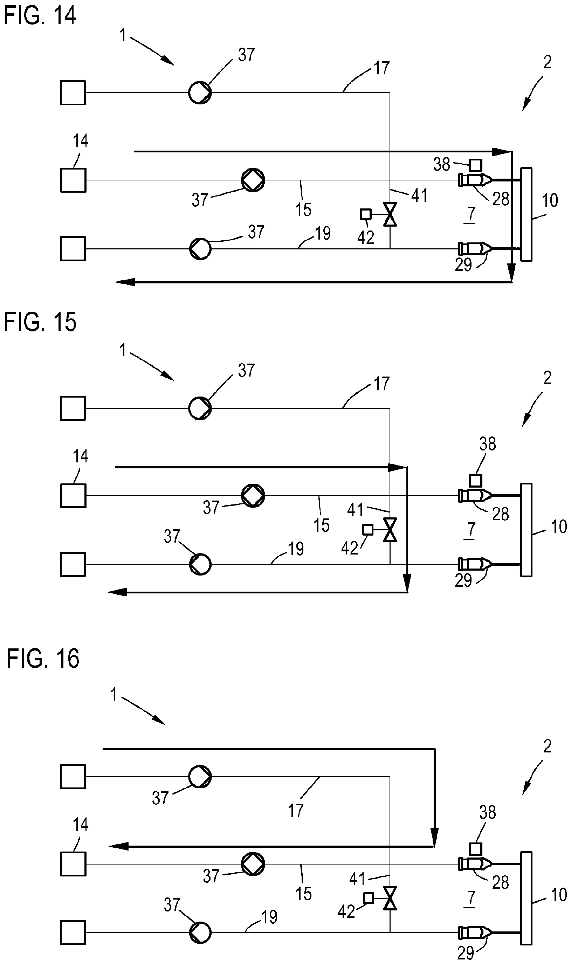

Ersichtlich mündet in den ersten Leitungsabschnitt 15b der Zuleitung 15 eine mit einer WC-seitigen Spüleinrichtung 43 (vgl. Fig. 14 ff) verbundene Frischwasserzuleitung 17. Sowohl vor als auch nach dem Mündungsbereich der Frischwasserzuleitung 17 in die Zuleitung 15 ist in den ersten Leitungsabschnitt 15a der Zuleitung 15, also zuleitungsseitig, eine Ventileinrichtung 18, z. B. in Form eines Magnetventils, geschaltet. Auch frischwasserzuleitungsseitig ist dem Mündungsbereich eine entsprechende Ventileinrichtung 18 vorgeschaltet.

Anhand der Fig. 2, 3 ist weiter zu erkennen, dass von der Vorrichtung 2, d. h. insbesondere von der in dem Gehäuseteil 6 angeordneten vorrichtungsseitigen Zuführ- und Abführeinrichtung 7, eine Ableitung 19 in den durch die inneren Flächenabschnitte 4b des Grundkörper 2 begrenzten Innenraum des WCs 3 führt. Derart ist es möglich, Urin nach der mittels der Vorrichtung 2 durchgeführten Analyse wieder in das WC 3 zurückzuführen und so einem WC-seitigen Abfluss zuzuführen. Bei der Ableitung 19 kann es sich ebenso um einen Laborschlauch handeln.

Anhand der Fig. 4, 5 soll die lösbare Befestigung des vorrichtungsseitigen Gehäuseteils 6 an dem WC-seitigen Grundkörper 4 erläutert werden. Fig. 4 zeigt den WC-seitigen Grundkörper 4 ohne an diesem befestigtes Gehäuseteil 6, Fig. 5 zeigt den WC-seitigen Grundkörper 4 mit an diesem befestigten Gehäuseteil 6. Wie anhand von Fig. 4 ersichtlich ist, ist der Grundkörper 4 im Bereich der freiliegenden vorderen Flächenabschnitte 4d mit Befestigungselementen 20 versehen. Die Befestigungselemente 20 sind z. B. mittels einer Klebeverbindung an dem Grundkörper 4 befestigt. Die Befestigungselemente 20 umfassen nicht näher bezeichnete, Gewinde aufweisende bolzenförmige Befestigungsabschnitte, über welche sich das Gehäuseteil 6 an diesen kraftschlüssig, d. h. insbesondere mittels einer Schraubverbindung, befestigen lässt. Selbstverständlich weist das Gehäuseteil 6 entsprechende Gewindeaufnahmen oder Durchbrechungen zur Realisierung der Befestigung, d. h. insbesondere der Schraubverbindung, auf. Typischerweise sind grundkörperseitig drei entsprechende Befestigungselemente 20 vorgesehen, so dass sich eine Drei-Punkt-Befestigung des Gehäuseteils 6 an dem Grundkörper 4 verwirklichen lässt.

Fig. 6 zeigt eine Prinzipdarstellung einer Entnahmeeinrichtung 14 der bzw. einer Sanitäreinrichtung 1 gemäß einem Ausführungsbeispiel der Erfindung. Die in Fig. 6 in einer Einzelansicht gezeigte Entnahmeeinrichtung 14 umfasst ein eine Öffnung des Grundkörpers 4 durchsetzendes hohlzylindrisches Rohrelement 21, welches relativ zu einem grundkörperseitig fest montierten, scheibenförmigen Schließelement 22 zwischen einer Öffnungs- und einer Schließstellung bewegbar gelagert ist. Die Bewegbarkeit des Rohrelements 21 zwischen der Öffnungs- und der Schließstellung ist in Fig. 6 durch den Doppelpfeil angedeutet.

In der in Fig. 6 gezeigten Öffnungsstellung, ist das Rohrelement 21 mit einem trichterförmigen Abschnitt, welcher gleichermaßen das freie Ende des Rohrelements 21 bildet, derart von dem Schließelement 22 weg bewegt, dass Urin aus dem Inneren des Grundkörpers 4 in bzw. durch das Rohrelement 21 strömen und somit in den sich an die Entnahmeeinrichtung 14 anschließenden ersten Leitungsabschnitt 15a der Zuleitung 15 in die Zuführ- und Abführeinrichtung 7 gelangen kann. Hierfür weist das Rohrelement 21 an oder im Bereich seines dem Grundkörper 4 abgewandten freien Endes ein Anschlussmittel (nicht gezeigt) zum Anschluss an die in die Zuführ- und Abführeinrichtung 7 führende Zuleitung 15 auf. In dem Anschlussmittel oder dem Anschlussmittel vorgeschaltet kann ein Sieb vorhanden sein.

Das Rohrelement 21 lässt sich in der Schließstellung durch eine dichtende Anlage an dem Schließelement 22 dichtend verschließen. Mit anderen Worten ist das Rohrelement 21 in der Schließstellung dichtend gegen das Schließelement 22 bewegt. Die dichtende Anlage wird durch ein außenumfangsseitig an dem Schließelement 22 angeordnetes, nicht näher bezeichnetes Dichtelement, z. B. in Form eines O-Rings, unterstützt.

Bewegungen des Rohrelements 21 werden über eine mit diesem gekoppelte Antriebseinrichtung 23 realisiert, über welche das Rohrelement 21, insbesondere zwischen der Öffnungs- und der Schließstellung, bewegbar ist. Die Antriebseinrichtung 23 umfasst hierfür z. B. einen magnetischen Antrieb in Form eines mit dem Rohrelement 21 über ein Ritzel- bzw. Zahnradelement 24 gekoppelten Drehmagnets. Zur Bewegung des Rohrelements 21 in die Schließstellung oder zur Unterstützung der Bewegung des Rohrelements 21 in die Schließstellung können Federmittel (nicht gezeigt) vorgesehen sein, über welche das Rohrelement 21 mit einer dieses in die Schließstellung bewegenden oder in der Schließstellung sichernden Federkraft beaufschlagbar ist.

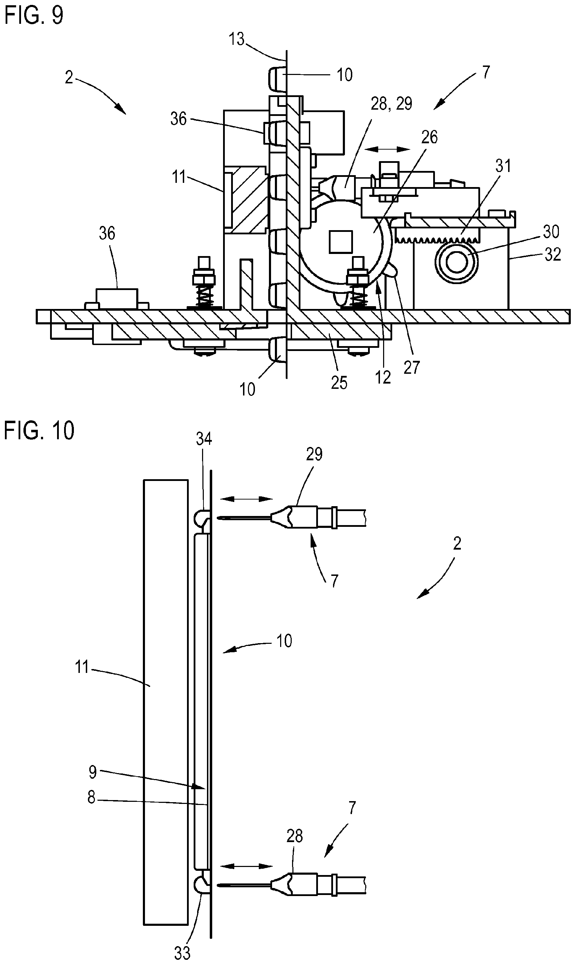

Die Fig. 7 - 10 zeigen jeweils eine Prinzipdarstellung einer Vorrichtung 2 zur Analyse von Urin gemäß einem Ausführungsbeispiel der Erfindung. Dabei zeigen die Fig. 7, 8 jeweils Schnittansichten unterschiedlicher Ausführungsbeispiele einer WC-seitig befestigten Vorrichtung 2, Fig. 9 eine vergrößerte Teilansicht der Vorrichtung 2 gemäß dem in Fig. 7 gezeigten Ausführungsbeispiel und Fig. 10 eine vergrößerte Teilansicht einer Aufsicht auf die Vorrichtung 2.

Hinsichtlich des in Fig. 7 gezeigten Ausführungsbeispiels ist anzumerken, dass das vorrichtungsseitige Gehäuseteil 6 im Vergleich zu der in Fig. 3 gezeigten Darstellung eine geringfügig andere geometrisch-räumliche Gestalt aufweist, was grundsätzlich auf einen möglichen Gestaltungsspielraum der Vorrichtung 1 respektive der dieser zugehörigen Bestandteile schließen lässt.

Der wesentliche Unterschied zwischen den in den Fig. 7, 8 gezeigten unterschiedlichen Ausführungsbeispielen der Vorrichtung 2 besteht in der Anzahl der der Fördereinrichtung 12 zugehörigen Förderrollen. Während bei dem in Fig. 7 gezeigten Ausführungsbeispiel lediglich eine Fördereinrichtung 12 mit einer einzigen Förderrolle, von welcher ein entsprechender Verbund 13 mehrerer band- oder gurtförmig miteinander verbundener Urinteststreifen 10 abrollbar ist, vorhanden ist, ist bei dem in Fig. 8 gezeigten Ausführungsbeispiel eine Fördereinrichtung 12 mit zwei Förderrollen sowie diesen vor- bzw. nachgeschalteten, in dem Ausführungsbeispiel im Vergleich zu den Förderrollen kleineren Umlenkrollen vorgesehen. Entsprechend ist es bei dem in Fig. 8 gezeigten Ausführungsbeispiel möglich, dass ein Verbund 13 mehrerer band- oder gurtförmig miteinander verbundener Urinteststreifen 10 von einer ersten Förderrolle derart abrollbar ist, dass wenigstens ein in den Erfassungsbereich der Erfassungseinrichtung 11 zu fördernder Urinteststreifen 10 in den Erfassungsbereich bewegbar oder bewegt ist, und auf eine zweite Förderrolle derart aufrollbar ist, dass er aus dem Erfassungsbereich bewegbar oder bewegt ist.

Bei dem in Fig. 7 gezeigten Ausführungsbeispiel ist eine der Erfassungseinrichtung 11 nachgeschaltete Abtrenneinrichtung 25 vorgesehen (vgl. Fig. 9), welche zur Abtrennung wenigstens eines Urinteststreifens 10 aus dem Verbund 13 mehrerer band- oder gurtförmig miteinander verbundener Urinteststreifen 10 eingerichtet ist. Mittels der Abtrenneinrichtung 25 können einzelne oder mehrere verbrauchte Urinteststreifen 10 aus dem Verbund 13 gelöst und somit gesondert gehandhabt, d. h. insbesondere entsorgt, werden. Die Abtrenneinrichtung 25 umfasst hierfür z. B. eine nicht näher bezeichnete Schneide oder einen Laser.

Aus dem Verbund 13 abgetrennte Urinteststreifen 10 können in einem Aufnahmeabteil 35, welches zur Aufnahme von verbrauchten, d. h. insbesondere zwischenzeitlich mit einer bestimmten Urinmenge befüllten Urinteststreifen 10, ausgebildet und lösbar an dem Gehäuseteil 6 befestigt ist, aufgenommen und gesammelt werden.

In Fig. 9 ist eine vergrößerte Ansicht des in Fig. 7 gezeigten Ausführungsbeispiels der Vorrichtung 2 gezeigt. Ersichtlich erstreckt sich hier ein entsprechender Verbund 13 miteinander verbundener Urinteststreifen 10 zwischen einem zwischen der Erfassungseinrichtung 11 und der Zuführ- und Abführeinrichtung 7 gebildeten Spaltraum und somit durch einen entsprechenden Erfassungsbereich der Erfassungseinrichtung 11. Die Führung und Positionierung des Verbunds 13 wird durch ein ebenso einen Teil der Fördereinrichtung 12 bildendes, drehbar gelagertes Noppenrad 26 unterstützt. Das Noppenrad 26 umfasst durch umfangsmäßig beabstandet angeordnete, sich radial erstreckende Noppen 27 ausgebildete Förderabschnitte zur Förderung wenigstens eines Urinteststreifens 10. Durch Rotation des Noppenrads 26 können die Urinteststreifen 10 kontinuierlich oder diskontinuierlich durch den Erfassungsbereich befördert werden.

Anhand der Fig. 9, 10 ist erkennbar, dass die die Zuführ- und Abführeinrichtung 7 ein linear bzw. translatorisch bewegbar gelagertes, kanülenartiges Zuführelement 28 und ein entsprechend bewegbar gelagertes, kanülenartiges Abführelement 29 umfasst. Das Zuführelement 28 dient zum Zuführen einer bestimmten Urinmenge in einen Zuführbereich 33 einer Analysekammer 9 eines Urinteststreifens 10, das Abführelement 29 dient zum Abführen einer bestimmten Urinmenge aus einem Abführbereich 34 einer Analysekammer 9 eines Urinteststreifens 10.

Zur Realisierung von Bewegungen des Zuführelements 28 und des Abführelements 29 weist die Zuführ- und Abführeinrichtung 7 eine mit diesen gekoppelte Antriebseinrichtung 32 auf. Die Antriebseinrichtung 32 umfasst einen Elektromotor (nicht gezeigt). Über die Antriebseinrichtung 32 ist das Zuführ- bzw. Abführelement 28, 29 derart gegen den oder einen urinteststreifenseitigen Zuführbereich 33 bzw. Abführbereich 34 bewegbar, dass eine kanülenartige Spitze des Zuführ- bzw. Abführelements 28, 29 zur Zu- und/oder Abführung einer bestimmten Urinmenge in die oder aus der urinteststreifenseitigen Analysekammer 9 in einen urinteststreifenseitigen Zu- und/oder Abführbereich 33, 34 eindringt. Die bewegbare Lagerung und somit die Bewegungsachse des Zuführelements 28 und des Abführelements 29 sind durch den Doppelpfeil angedeutet.

Ersichtlich ist die Antriebseinrichtung 32 über mehrere Bauteile, wie z. B. ein Zahnrad 30 und eine mit diesem kämmende Zahnstange 31, mit dem Zuführ- und dem Abführelement 28, 29 gekoppelt, so dass sich rotatorische Bewegungen einer antriebseinrichtungsseitigen Abtriebswelle (nicht gezeigt) über das Zahnrad 30 und die Zahnstange 31 in translatorische Bewegungen des Zuführelements 28 und des Abführelements 29 umsetzen lassen.

Die Vorrichtung 2 ist ferner mit einer oberhalb der Erfassungseinrichtung 11 angeordneten Positionsermittlungseinrichtung 36 ausgestattet, welche dazu eingerichtet ist, eine im Hinblick auf die Erfassung eines entsprechenden optisch erfassbaren Parameters eines urinteststreifenseitigen Analysebereichs 8 respektive die Erfassung einer Änderung eines solchen mittels der Erfassungseinrichtung 11 korrekte Positionierung eines Urinteststreifens 10 zu ermitteln. Eine weitere, unterhalb der Erfassungseinrichtung 11 angeordnete Positionsermittlungseinrichtung 36 ist dazu eingerichtet, eine Ausgangsstellung der Abtrenneinrichtung 25 zu ermitteln. Die Ausgangsstellung der Abtrenneinrichtung 25 definiert sich dadurch, dass in dieser kein Abtrennen eines Urinteststreifens 10 aus dem Verbund 13 erfolgt. Die Positionsermittlungseinrichtungen 36 umfassen für die genannten Zwecke jeweils Lichtschranken.

Wenngleich in den Fig. 7 - 10 nicht ersichtlich, umfasst die Vorrichtung 2 ferner eine in eine in ein Zuführelement 28 führende Zuleitung 15 geschaltete Pumpeneinrichtung 37 und eine in eine aus einem Abführelement 29 führende Ableitung 19 geschaltete Pumpeneinrichtung 37. Die Pumpeneinrichtungen 37 sind jedoch in den Fig. 14 ff schematisch dargestellt. Die Pumpeneinrichtungen 37, bei welchen es sich z. B. um kleinbauende Drehkreiselpumpen handeln kann, dienen insbesondere dazu, Urin durch die Vorrichtung 2 zu befördern, d. h. insbesondere in eine entsprechende urinteststreifenseitige Analysekammer 9 zu fördern bzw. zu pumpen respektive aus einer entsprechenden urinteststreifenseitigen Analysekammer 9 zu fördern bzw. zu pumpen.

Ebenso ist die Vorrichtung 2 mit einer Druckermittlungseinrichtung 38, welche zur Ermittlung des Druckes einer in den urinteststreifenseitigen Zuführbereich 33 geführten Urinmenge, insbesondere des Druckes der durch das Zuführelement 28 geführten Urinmenge eingerichtet ist, ausgestattet. Die Druckermittlungseinrichtung 38 umfasst hierfür geeignete Drucksensoren. Die Druckermittlungseinrichtung 38 ist ebenso in den Fig. 14 ff schematisch dargestellt. Anhand des über eine solche Druckermittlungseinrichtung 38 ermittelbaren Drucks einer in den urinteststreifenseitigen Zuführbereich 33 geführten Urinmenge lassen sich Rückschlüsse auf etwaig vorhandene Gasblasen, insbesondere Luftblasen, innerhalb der Urinmenge ziehen, welche die Aussagekraft der Erfassungsinformation gegebenenfalls beinträchtigen können.

Die Vorrichtung 2 umfasst ferner eine in den Fig. 14 ff schematisch gezeigte Bypassleitung 41, welche die in ein Zuführelement 28 führende Zuleitung 15 und eine aus einem Abführelement 29 führende Ableitung 19 verbindet. In die Bypassleitung 41 ist eine Ventileinrichtung 42 zum Öffnen und Schließen der Bypassleitung 41 geschaltet.

Fig. 10 zeigt eine vergrößerte Teildarstellung einer Aufsicht auf die Vorrichtung 2. Anhand von Fig. 10 ist die bewegbare Lagerung des der Zuführ- und Abführeinrichtung 7 zugehörigen Zuführ- und Abführelements 28, 29 wiederum durch einen Doppelpfeil angedeutet. In der in Fig. 10 gezeigten Darstellung sind die Zuführ- und Abführelemente 28, 29, welche typischerweise gleichzeitig und gleichförmig angetrieben bzw. bewegt werden, von dem Urinteststreifen 10 respektive einem entsprechenden urinteststreifenseitigen Zuführbereich 33 und einem urinteststreifenseitigen Abführbereich 34 beabstandet. Eine Zu- bzw. Abführung einer bestimmten Urinmenge in die urinteststreifenseitige Analysekammer 9 ist erst möglich, wenn das Zuführelement 28 in den urinteststreifenseitigen Zuführbereich 33 und das Abführelement 29 in den urinteststreifenseitigen Abführbereich 34 eingedrungen ist. Ersichtlich definiert sich der urinteststreifenseitige Zuführbereich 33 wie auch der urinteststreifenseitige Abführbereich 34 konstruktiv durch eine kalotten- bzw. kugelsegmentförmige Ausbuchtung.

Der weitere Aufbau eines einen Teil einer Urinteststreifeneinrichtung 39 bildenden Urinteststreifens 10 wird im Weiteren mit Bezug auf die Fig. 11 - 13, welche jeweils eine Prinzipdarstellung einer Urinteststreifeneinrichtung 39 gemäß einem Ausführungsbeispiel der Erfindung, zeigen, beschrieben. Dabei zeigt Fig. 11 einen einzelnen Urinteststreifen 10 in einer perspektivischen Ansicht, Fig. 12 eine Schnittansicht entlang der in Fig. 11 gezeigten Schnittlinien XII - XII und Fig. 13 eine Aufsicht auf einen Verbund 13 mehrerer band- oder gurtförmig miteinander verbundener Urinteststreifen 10.

Anhand von Fig. 11 ist ersichtlich, dass ein einzelner Urinteststreifen 10 typischerweise eine rechteckige Grundform aufweist. Eine einen Analysebereich 8 zur Analyse von Urin aufweisende Analysekammer 9 befindet sich zwischen einem oberen Kapselungselementabschnitt 40a und einem unteren Kapselungselementabschnitt 40b. Der obere Kapselungselementabschnitt 40a und der untere Kapselungselementabschnitt 40b bilden gemeinsam ein Kapselungselement 40, über welches der Analysebereich 8 gekapselt und so vor äußeren Einflüssen, d. h. insbesondere vor Feuchtigkeit, geschützt ist.

Der obere Kapselungselementabschnitt 40a ist als dreidimensional komplex geformtes Formteil ausgebildet und weist einen im Wesentlichen ebenen, die Analysekammer 9 begrenzenden mittleren Wandabschnitt und zwei kalotten- oder kugelsegmentförmig gewölbte, den Zuführbereich 33 und den Abführbereich 34 begrenzende seitliche Wandabschnitte auf. Selbstverständlich ist ein Durchgang zwischen dem Zu- und Abführbereich 33, 34 und der Analysekammer 8 gegeben, welche sonach miteinander kommunizieren.

Der obere Kapselungselementabschnitt 40a ist aus einem transparenten Kunststoffmaterial, wie z. B. PC, gebildet. Das Kunststoffmaterial ist dermaßen transparent, dass es eine Erfassung einer Veränderung eines optisch erfassbaren Parameters des Analysebereichs 8, d. h. z. B. einen Farbumschlag, mittels der Erfassungseinrichtung 11 zulässt.

Der untere Kapselungselementabschnitt 40b ist nicht dreidimensional komplex geformt, sondern flächig bzw. folienartig ausgebildet. Der untere Kapselungselementabschnitt 40b liegt entsprechend als Flächenteil bzw. als Folie vor. Seine Dicke liegt typischerweise in einem Bereich zwischen 30 und 120 µm, insbesondere in einem Bereich zwischen 70 und 110µm.

Der untere Kapselungselement 40b ist aus einem elastischen Kunststoffmaterial, wie z. B. PE und/oder PET, bzw. einem Kunststoffverbundmaterial gebildet. Im Hinblick auf die elastischen Eigenschaften kann der untere Kapselungselementabschnitt 40b auch aus einem thermoplastischen Elastomer, d. h. z. B. TPO und/oder TPV, gebildet sein.

Die elastischen Eigenschaften des unteren Kapselungselementabschnitts 40b ermöglichen es, dass ein in den Zuführ- bzw. Abführbereich 33, 34 eindringendes, diesen perforierendes Zuführ- bzw. Abführelement 28, 29 abschnittsweise dichtend umschließbar ist. Der untere Kapselungselementabschnitt 40b schmiegt sich also an ein in den Zuführ- bzw. Abführbereich 33, 34 eindringendes bzw. eingedrungenes Zuführ- bzw. Abführelement 28, 29 an, so dass es sichergestellt ist, dass während des Zuführens bzw. Abführen einer bestimmten Urinmenge in den bzw. aus dem Urinteststreifen 10 keine Leckage auftritt.

Die elastischen Eigenschaften des unteren Kapselungselementabschnitts 40b ermöglichen es gleichermaßen, dass ein perforierter Bereich durch die diesen begrenzenden Materialabschnitte dichtend verschlossen ist bzw. wird. Der untere Kapselungselementabschnitt 40b dient sonach auch als Septum bzw. als Verschlussmembran, wodurch sichergestellt ist, dass auch nach dem Zuführen bzw. Abführen einer bestimmten Urinmenge aus dem Urinteststreifen 10 keine Leckage auftritt.

Fig. 13 zeigt einen Verbund 13 mehrerer Urinteststreifen 10. Die Urinteststreifen 10 sind dabei band- oder gurtförmig miteinander verbunden. Der Verbund 13 kann sonach als mehrere Urinteststreifen 10 aufweisendes Band bzw. mehrere Urinteststreifen 10 aufweisender Gurt bezeichnet bzw. erachtet werden. Ersichtlich sind die Urinteststreifen 10 im Bereich ihrer jeweiligen Längsseiten miteinander verbunden. Die Verbindung zwischen entsprechenden Urinteststreifen 10 ist dabei insbesondere über jeweilige Kapselungselemente 40 gebildet, welche unter Ausbildung kontinuierlicher oder diskontinuierlicher Verbindungsbereiche zumindest abschnittsweise miteinander verbunden sind. Die Kapselungselemente 40 jeweils benachbart angeordneter Urinteststreifen 10 können dabei z. B. verklebt oder verschweißt sein.

Anhand der Fig. 14 - 19, welche jeweils eine schematische Darstellung unterschiedlicher Betriebsmodi einer Sanitäreinrichtung 1 gemäß einem Ausführungsbeispiel der Erfindung zeigen, werden im Weiteren unterschiedliche Ausführungsbeispiele eines Verfahrens zum Betrieb einer Sanitäreinrichtung 1 mit einer dieser zugehörigen Vorrichtung 2 beschrieben. Die Beschreibung der Betriebsmodi basiert auf einer wie oben beschriebenen, und in den Fig. 14 ff jeweils dargestellten Konfiguration der Sanitäreinrichtung 1 bzw. der Vorrichtung 2. Fluidströmungen sind in den Fig. 14 ff allgemein durch Pfeile angedeutet. Eine spüleinrichtungsseitige Pumpeneinrichtung ist ebenso mit Bezugszeichen 37 versehen.

In Fig. 14 ist ein beispielhafter Betriebsmodus zur Realisierung einer Entnahme einer bestimmten Urinmenge und einer Analyse dieser, d. h. insbesondere einer Erfassung einer entsprechenden Erfassungsinformation, gezeigt. Die über die Entnahmeeinrichtung 14 entnommene Urinmenge wird mittels der Pumpeneinrichtungen 37 über die Zuleitung 15 in den Urinteststreifen 10, d. h. über den Analysebereich 8 und somit durch die Analysekammer 9, gefördert bzw. gepumpt. Die Urinmenge wird im Weiteren, d. h. insbesondere nach der Analyse und Erzeugung der Erfassungsinformation, über die Ableitung 19 aus dem Urinteststreifen 10 und aus der Vorrichtung 2 gefördert bzw. gepumpt. Selbstverständlich sind entsprechende vorrichtungsseitige Zuführelemente 28 bzw. Abführelemente 29 zuvor in entsprechende urinteststreifenseitige Zuführ- bzw. Abführbereiche 33, 34 eingedrungen. Die in die Bypassleitung 41 geschaltete Ventileinrichtung 42 ist hierbei geschlossen. Die Erfassungseinrichtung 11 erzeugt eine entsprechende Erfassungsinformation, welche, wie im Weiteren beschrieben wird, insbesondere an eine in die Vorrichtung 2 integrierte oder an eine externe, d. h. räumlich getrennt zu der Vorrichtung 2 angeordnete, Auswertungseinrichtung (nicht gezeigt) übermittelt wird.

In Fig. 15 ist ein beispielhafter Betriebsmodus zur Realisierung eines Entleerens der Zuleitung 15 und der Ableitung 19 gezeigt. Die in die Bypassleitung 41 geschaltete Ventileinrichtung 42 ist hierbei geöffnet, so dass in der Zuleitung 15 und der Ableitung 19 gegebenenfalls vorhandene Urinmengenreste abgepumpt werden können.

In Fig. 16 ist ein beispielhafter Betriebsmodus zur Realisierung eines Spülens der Zuleitung 15 wie auch der dieser vorgeschalteten Entnahmeeinrichtung 14 gezeigt. Ersichtlich wird hierbei von einer WC-seitigen Spüleinrichtung 43 über die Frischwasserzuleitung 17 bereitgestelltes Spülfluid, insbesondere Wasser, durch die Zuleitung 15 wie auch durch die Entnahmeeinrichtung 14 gefördert bzw. gepumpt. Die Ventileinrichtungen 18 wie auch die in die Bypassleitung 41 geschaltete Ventileinrichtung 42 sind hierbei derart geschaltet, dass ein Einströmen des Spülfluids weder in die Zuführ- und Abführeinrichtung 7 noch in die Ableitung 19 möglich ist.

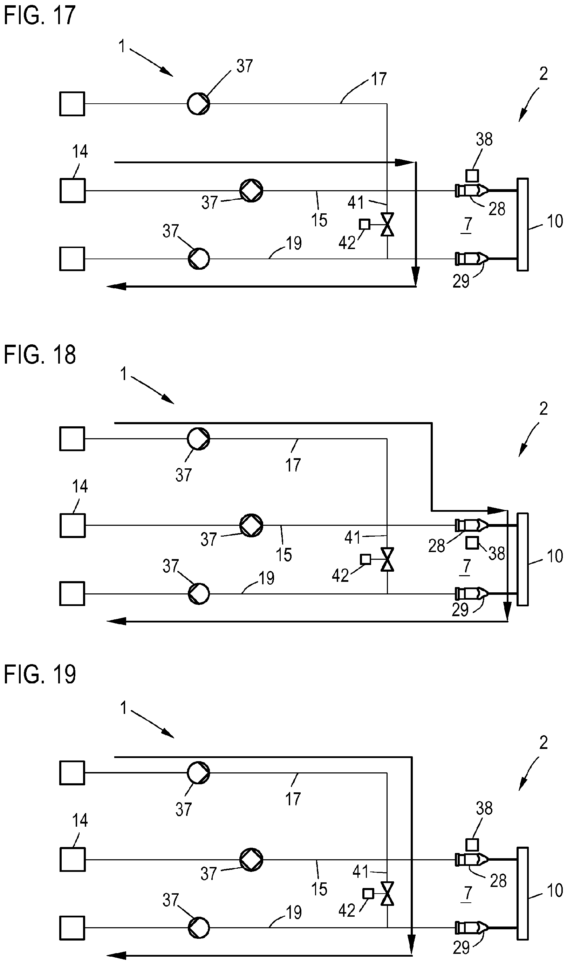

In Fig. 17 ist ein beispielhafter Betriebsmodus zur Realisierung eines dem Spülen der Zuleitung 15 folgenden Entleerens der Zuleitung 15 wie auch der dieser vorgeschalteten Entnahmeeinrichtung 14 gezeigt. Das Entleeren der Zuleitung 15 wie auch der Entnahmeeinrichtung 14 erfolgt durch Öffnen der in die Bypassleitung 41 geschalteten Ventileinrichtung 42, so dass Spülfluidreste über die Ableitung 19 aus der Zuleitung 15 und der Entnahmeeinrichtung 14 gefördert bzw. gepumpt werden können.

In Fig. 18 ist ein beispielhafter Betriebsmodus zur Realisierung eines Spülens der Zuführ- und Abführeinrichtung 7 sowie eines Urinteststreifens 10 gezeigt. Ersichtlich wird hierbei von der WC-seitigen Spüleinrichtung 43 über die Frischwasserzuleitung 17 bereitgestelltes Spülfluid, insbesondere Wasser, durch die Zuführ- und Abführeinrichtung 7 wie auch den Urinteststreifen 10, d. h. insbesondere durch die urinteststreifenseitige Analysekammer 9, gefördert bzw. gepumpt. Die Ventileinrichtungen 18 wie auch die in die Bypassleitung 41 geschaltete Ventileinrichtung 42 sind hierbei derart geschaltet, dass ein Einströmen des Spülfluids in die Zuleitung 15 nicht möglich ist.

Schließlich zeigt Fig. 19 einen beispielhaften Betriebsmodus zur Realisierung eines Spülens der Ableitung 19, welches dem Spülen der Zuführ- und Abführeinrichtung 7 sowie des Urinteststreifens 10 folgen kann. Ersichtlich wird hierbei von der WC-seitigen Spüleinrichtung 43 über die Frischwasserzuleitung 17 bereitgestelltes Spülfluid, insbesondere Wasser, durch die Ableitung 19 gefördert bzw. gepumpt. Die Ventileinrichtungen 18 wie auch die in die Bypassleitung 41 geschaltete Ventileinrichtung 42 sind hierbei derart geschaltet, dass ein Einströmen des Spülfluids weder in die Zuleitung 15 noch in die Zuführ- und Abführeinrichtung 7 möglich ist.

Der Betrieb der Sanitäreinrichtung 1 respektive der Vorrichtung 2, d. h. insbesondere auch die Durchführung der mit Bezug auf die Fig. 14 - 19 beschriebenen Betriebsmodi, werden durch eine zentrale Steuerungseinrichtung (nicht gezeigt) gesteuert.

Die Steuerungseinrichtung kommuniziert hierfür insbesondere mit allen vorrichtungsseitigen Einrichtungen. In der Steuerungseinrichtung ist typischerweise wenigstens eine Steuerungsvorschrift hinterlegt, gemäß welcher eine konzertierte, d. h. aufeinander abgestimmte Steuerung des Betriebs der Zuführ- und Abführeinrichtung 7, der Erfassungseinrichtung 11 sowie weiterer der Vorrichtung 2 zugehöriger Einrichtungen möglich ist. Zu Letzteren zählen insbesondere entsprechende Druckermittlungseinrichtungen 38, Pumpeneinrichtungen 37 sowie die Ventileinrichtungen 18, 42.

Die Erfassungseinrichtung 11 ist typischerweise zur Kommunikation mit wenigstens einer Auswertungseinrichtung, welche zur Auswertung einer von der Erfassungseinrichtung erzeugten Erfassungsinformation und zur Ermittlung einer eine Analyse der auf dem Analysebereich des Urinteststreifens befindlichen Urinmenge beschreibenden Auswertungsinformation eingerichtet ist, eingerichtet. Wie erwähnt, kann die Auswertungseinrichtung Teil der Vorrichtung 2 oder extern sein. In letzterem Fall umfasst die Vorrichtung 2 eine der Erfassungseinrichtung 11 zugeordnete Sende- und/oder Empfangseinrichtung (nicht gezeigt), welche eine drahtgebundene oder drahtlose Übermittlung entsprechender Erfassungsinformationen ermöglicht. Die Sanitäreinrichtung 1 respektive die Vorrichtung 2 ist sonach, z. B. mittels Bluetooth, WLAN etc., an ein lokales oder globales Datennetzwerk, d. h. z. B. an ein lokales Intranet oder das Internet, angeschlossen bzw. in ein solches eingebunden. Entsprechend können Erfassungsinformationen an entsprechende Auswertungseinrichtungen übermittelt werden, in welchen auf Basis der Erfassungsinformationen Rückschlüsse auf die anteilsmäßige bzw. chemische Zusammensetzung der auf den Analysebereich aufgebrachten Urinmenge gezogen werden können.

## Patentansprüche

1. Vorrichtung (2) zur Analyse von Urin, umfassend:
- eine Zuführ- und Abführeinrichtung (7), welche zur Zuführung einer bestimmten Urinmenge in eine wenigstens einen Analysebereich (8) aufweisende Analysekammer (9) eines Urinteststreifens (10) und zur Abführung einer bestimmten Urinmenge aus einer wenigstens einen Analysebereich (8) aufweisenden Analysekammer (9) eines Urinteststreifens (10) eingerichtet ist, wobei die Zuführ- und Abführeinrichtung (7) wenigstens ein bewegbar gelagertes Zuführ- und/oder Abführelement (28, 29) zum Zuführen einer bestimmten Urinmenge in einen Zuführbereich (33) der Analysekammer (9) des Urinteststreifens (10) und/oder zum Abführen einer bestimmten Urinmenge aus einem Abführbereich (34) der Analysekammer (9) des Urinteststreifens (10) aufweist, und
- eine Erfassungseinrichtung (11), welche zur Erfassung einer zumindest abschnittsweisen Änderung wenigstens eines optisch erfassbaren Parameters, welcher sich in Abhängigkeit der Zusammensetzung einer den Analysebereich (8) kontaktierenden Urinmenge optisch erfassbar verändert, des wenigstens einen entsprechenden Analysebereichs (8) des oder eines entsprechenden Urinteststreifens (10) sowie zur Erzeugung einer Erfassungsinformation, welche wenigstens einen optisch erfassten Parameter des oder eines entsprechenden Analysebereichs (8) oder eine Änderung eines solchen beschreibt, eingerichtet ist.

2. Vorrichtung nach Anspruch 1, **dadurch gekennzeichnet, dass** die Zuführ- und Abführeinrichtung (7) wenigstens eine mit dem wenigstens einen Zuführ- und/oder Abführelement (28, 29) gekoppelte Antriebseinrichtung (32) aufweist, über welche das wenigstens eine Zuführ- und/oder Abführelement (28, 29) derart gegen den oder einen urinteststreifenseitigen Zu- und/oder Abführbereich (33, 34) bewegbar ist, dass eine, insbesondere kanülenartige, Spitze des Zuführ- und/oder Abführelements (28, 29) zur Zu- und/oder Abführung der oder einer bestimmten Urinmenge in die oder aus der urinteststreifenseitigen Analysekammer (9) in den urinteststreifenseitigen Zu- und/oder Abführbereich (33, 34) eindringt.

3. Vorrichtung nach einem der vorangehenden Ansprüche, **gekennzeichnet durch** wenigstens eine Fördereinrichtung (12), welche zur Förderung wenigstens eines Urinteststreifens (10) in einen vorrichtungsseitig definierten Erfassungsbereich, in welchem mittels der Erfassungseinrichtung (11) ein Erfassen einer zumindest abschnittsweisen Änderung des wenigstens einen optisch erfassbaren Parameters möglich ist, und/oder aus dem oder einem solchen Erfassungsbereich eingerichtet ist.

4. Vorrichtung nach Anspruch 3, **dadurch gekennzeichnet, dass** die Fördereinrichtung (12) wenigstens ein drehbar gelagertes Förderelement mit Förderabschnitten zur Förderung wenigstens eines Urinteststreifens (10, insbesondere ein Noppenrad (26) mit durch umfangsmäßig beabstandet angeordnete Noppen ausgebildeten Förderabschnitten zur Förderung wenigstens eines Urinteststreifens (10), und/oder wenigstens ein Förderband mit Förderabschnitten zur Förderung wenigstens eines Urinteststreifens (10) und/oder wenigstens eine Förderrolle, von welcher ein Verbund (13) mehrerer band- oder gurtförmig miteinander verbundener Urinteststreifen (10) abrollbar oder auf welche ein Verbund (13) mehrerer band- oder gurtförmig miteinander verbundener Urinteststreifen (10) aufrollbar ist, umfasst oder als solche ausgebildet ist.

5. Vorrichtung nach Anspruch 4, **dadurch gekennzeichnet, dass** die Fördereinrichtung (12) wenigstens zwei Förderrollen umfasst, wobei ein Verbund (13) mehrerer band- oder gurtförmig miteinander verbundener Urinteststreifen (10) von einer ersten Förderrolle derart abrollbar ist, dass wenigstens ein in den Erfassungsbereich zu fördernder Urinteststreifen (10) in den Erfassungsbereich bewegbar oder bewegt ist, und auf eine zweite Förderrolle derart aufrollbar ist, dass der wenigstens eine Urinteststreifen (10) aus dem Erfassungsbereich bewegbar oder bewegt ist.

6. Vorrichtung nach einem der vorangehenden Ansprüche, **gekennzeichnet durch** wenigstens eine der Erfassungseinrichtung (11) nachgeschaltete Abtrenneinrichtung (25), welche zur Abtrennung wenigstens eines Urinteststreifens (10) aus einem Verbund (13) mehrerer band- oder gurtförmig miteinander verbundener Urinteststreifen (10) eingerichtet ist.

7. Vorrichtung nach einem der vorangehenden Ansprüche, **gekennzeichnet durch** wenigstens ein Gehäuseteil (6), welches zur Aufnahme der Zuführ- und Abführeinrichtung (7), der Erfassungseinrichtung (11) sowie gegebenenfalls weiterer Einrichtungen der Vorrichtung (2) ausgebildet ist.

8. Vorrichtung nach einem der vorangehenden Ansprüche, **gekennzeichnet durch** wenigstens eine Bypassleitung (41), welche eine in ein Zuführelement (28) führende Zuleitung (15) und eine aus einem Abführelement (29) führende Ableitung (19) verbindet, wobei in die Bypassleitung (41) wenigstens eine Ventileinrichtung (42) zum Öffnen und Schließen der Bypassleitung (41) geschaltet ist.

9. Vorrichtung nach einem der vorangehenden Ansprüche, **dadurch gekennzeichnet, dass** die Erfassungseinrichtung (11) zur Kommunikation mit wenigstens einer Auswertungseinrichtung, welche zur Auswertung einer von der Erfassungseinrichtung (11) erzeugten Erfassungsinformation und zur Ermittlung einer eine Analyse der auf dem Analysebereich (8) des Urinteststreifens (10) befindlichen Urinmenge beschreibenden Auswertungsinformation eingerichtet ist, eingerichtet ist.

10. Vorrichtung nach einem der vorangehenden Ansprüche, **gekennzeichnet durch** eine Steuerungseinrichtung, welche zur Steuerung des Betriebs der Zuführ- und Abführeinrichtung (7), der Erfassungseinrichtung (11) sowie gegebenenfalls weiterer vorrichtungsseitiger Einrichtungen, insbesondere wenigstens einer Druckermittlungseinrichtung (38), wenigstens einer Pumpeneinrichtung (37), wenigstens einer in eine entsprechende Bypassleitung (41) geschalteten Ventileinrichtung (42) eingerichtet ist.

11. Sanitäreinrichtung (1), umfassend ein, insbesondere wandhängendes oder bodenstehendes, WC (3) mit einem, insbesondere keramischen, Grundkörper (4) und mit einer Vorrichtung (2) nach einem der Ansprüche 1 - 10.

12. Sanitäreinrichtung nach Anspruch 11, **gekennzeichnet durch** wenigstens eine in dem Grundkörper (4) ausgebildete oder angeordnete Entnahmeeinrichtung (14), umfassend wenigstens ein in einer Öffnung des Grundkörpers (4) angeordnetes Rohrelement (21), welches zwischen einer Öffnungs- und einer Schließstellung relativ zu einem Schließelement (22) bewegbar gelagert ist.

13. Sanitäreinrichtung nach Anspruch 12, **dadurch gekennzeichnet, dass** das Rohrelement (21) an oder im Bereich seines dem Grundkörper (4) abgewandten freien Endes ein Anschlussmittel zum Anschluss an die oder eine vorrichtungsseitige Zuführ- und Abführeinrichtung (7) aufweist.

14. Sanitäreinrichtung nach einem der Ansprüche 11 bis 13, **dadurch gekennzeichnet, dass** eine aus der oder einer vorrichtungsseitigen Zuführ- und Abführeinrichtung (7) führende Ableitung (19) in einen durch den Grundkörper (4) begrenzten Innenraum führt.

15. Sanitäreinrichtung nach einem der Ansprüche 11 bis 14, **gekennzeichnet durch** wenigstens eine Spüleinrichtung (43) zur Durchführung eines Spülvorgangs des WCs (3), wobei wenigstens eine spüleinrichtungsseitige Fluidleitung (17) mit der oder einer in eine vorrichtungsseitige Zuführ- und Abführeinrichtung (7) führenden Zuleitung (15) verbindbar oder verbunden ist.

## Claims

1. Apparatus (2) for analysis of urine, comprising:
- a delivery and discharge device (7), which is set up to deliver a defined quantity of urine to an analysis chamber (9) of a urine test strip (10) having at least one analysis region (8) and for discharging a defined quantity of urine from an analysis chamber (9) of a urine test strip (10) having at least one analysis region (8), wherein the delivery and discharge device (7) has at least one movably mounted delivery and/or discharge element (28, 29) for delivering a defined quantity of urine to a delivery region (33) of the analysis chamber (9) of the urine test strip (10) and/or for discharging a defined quantity of urine from a discharge region (34) of the analysis chamber (9) of the urine test strip (10), and
- a detection device (11), which is set up to detect an at least local change of at least one optically detectable parameter of the at least one corresponding analysis region (8) of the or a corresponding urine test strip (10), which parameter changes in an optically detectable manner as a function of the composition of a quantity of urine contacting the analysis region (8), and to generate detection information which describes at least one optically detected parameter of the or a corresponding analysis region (8) or which describes a change in such a parameter.

2. Apparatus according to Claim 1, **characterized in that** the delivery and discharge device (7) has at least one drive device (32) coupled to the at least one delivery and/or discharge element (28, 29), by means of which drive device (32) the at least one delivery and/or discharge element (28, 29) is movable towards the or a delivery and/or discharge region (33, 34) of the urine test strip in such a way that a tip, in particular a cannula-like tip, of the delivery and/or discharge element (28, 29) penetrates the delivery and/or discharge region (33, 34) of the urine test strip in order to deliver and/or discharge the or a defined quantity of urine into/from the urine test strip analysis chamber (9).

3. Apparatus according to one of the preceding claims, **characterized by** at least one conveying device (12), which is set up to convey at least one urine test strip (10) into a detection region defined on the apparatus side, in which detection region an at least local change of the at least one optically detectable parameter can be detected by means of the detection device (11), and/or which is set up to convey the at least one urine test strip (10) out of the detection region or one such detection region.

4. Apparatus according to Claim 3, **characterized in that** the conveying device (12) comprises, or is configured as, at least one rotatably mounted conveyor element with conveying sections for conveying at least one urine test strip (10), in particular a knobbed wheel (26) with conveying sections formed by knobs arranged a certain distance apart around the circumference for conveying at least one urine test strip (10), and/or at least one conveyor belt with conveying sections for conveying at least one urine test strip (10), and/or at least one conveying roller from which a set (13) of several urine test strips (10) interconnected like a band or belt can be unrolled or onto which a set (13) of several urine test strips (10) interconnected like a band or belt can be rolled.

5. Apparatus according to Claim 4, **characterized in that** the conveying device (12) comprises at least two conveying rollers, wherein a set (13) of several urine test strips (10) interconnected like a band or belt can be unrolled from a first conveying roller, in such a way that at least one urine test strip (10) to be conveyed into the detection region is movable or moved into the detection region, and can be rolled up onto a second conveying roller in such a way that the at least one urine test strip (10) is movable or moved out of the detection region.

6. Apparatus according to one of the preceding claims, **characterized by** at least one separation device (25) which is arranged downstream from the detection device (11) and which is set up to separate at least one urine test strip (10) from a set (13) of several urine test strips (10) interconnected like a band or belt.

7. Apparatus according to one of the preceding claims, **characterized by** at least one housing part (6), which is configured to hold the delivery and discharge device (7), the detection device (11) and, if appropriate, further devices of the apparatus (2).

8. Apparatus according to one of the preceding claims, **characterized by** at least one bypass line (41), with which a feed line (15) leading into a delivery element (28) is connected to a withdrawal line (19) leading from a discharge element (29), wherein at least one valve device (42) for opening and closing the bypass line (41) is installed in the bypass line (41).

9. Apparatus according to one of the preceding claims, **characterized in that** the detection device (11) is set up to communicate with at least one evaluation device which is set up to evaluate detection information generated by the detection device (11) and to acquire evaluation information describing an analysis of the quantity of urine located on the analysis region (8) of the urine test strip (10).

10. Apparatus according to one of the preceding claims, **characterized by** a control device, which is set up to control the operation of the delivery and discharge device (7), the detection device (11) and, if appropriate, further devices of the apparatus (2), in particular at least one pressure-determining device (38), at least one pump device (37), and at least one valve device (42) installed in a corresponding bypass line (41).

11. Sanitary facility (1) comprising a flush toilet (3), especially of the type suspended on a wall or floor-mounted, with a base body (4), especially made of ceramic, and with an apparatus (2) according to one of Claims 1-10.

12. Sanitary facility according to Claim 11, **characterized by** at least one removal device (14) formed or arranged in the base body (4), comprising at least one tubular element (21) which is arranged in an opening in the base body (4) and which is mounted movably relative to a closing element (22) between an open position and a closed position.

13. Sanitary facility according to Claim 12, **characterized in that** the tubular element (21) has, on or in the region of its free end directed away from the base body (4), an attachment means for attaching to the or a delivery and discharge device (7) of the apparatus.

14. Sanitary facility according to one of Claims 11 to 13, **characterized in that** a withdrawal line (19) leading from the or a delivery and discharge device (7) leads into an internal space delimited by the base body (4).

15. Sanitary facility according to one of Claims 11 to 14, **characterized by** at least one flushing device (43) for carrying out a flushing operation of the flush toilet (3), wherein at least one fluid line (17) of the flushing device is connectable or connected to the or a feed line (15) leading into a delivery and discharge device (7) of the apparatus.

## Revendications

1. Dispositif (2) d'analyse d'urine, comprenant :
- un dispositif d'alimentation et d'évacuation (7), qui est prévu pour acheminer une quantité d'urine déterminée dans une chambre d'analyse (9) d'une bandelette de test d'urine (10), présentant au moins une zone d'analyse (8), et pour évacuer une quantité d'urine déterminée hors d'au moins une chambre d'analyse (9) d'une bandelette de test d'urine (10), présentant une zone d'analyse (8), le dispositif d'alimentation et d'évacuation (7) présentant au moins un élément d'alimentation et/ou d'évacuation (28, 29) supporté de manière déplaçable pour acheminer une quantité d'urine déterminée dans une zone d'alimentation (33) de la chambre d'analyse (9) de la bandelette de test d'urine (10) et/ou pour évacuer une quantité d'urine déterminée hors d'une zone d'évacuation (34) de la chambre d'analyse (9) de la bandelette de test d'urine (10), et
- un dispositif de détection (11) qui est prévu pour détecter une variation au moins partielle d'au moins un paramètre détectable optiquement, qui varie de manière détectable optiquement, en fonction de la composition d'une quantité d'urine venant en contact avec la zone d'analyse (8) de l'au moins une zone d'analyse correspondante (8) de la ou d'une bandelette de test d'urine correspondante (10) et pour générer une information de détection qui décrit au moins un paramètre détecté optiquement de la ou d'une zone d'analyse correspondante (8) ou une variation d'un tel paramètre.

2. Dispositif selon la revendication 1, **caractérisé en ce que** le dispositif d'alimentation et d'évacuation (7) présente au moins un dispositif d'entraînement (32) accouplé à l'au moins un élément d'alimentation et/ou d'évacuation (28, 29), par le biais duquel l'au moins un élément d'alimentation et/ou d'évacuation (28, 29) peut être déplacé contre la ou une région d'alimentation et/ou d'évacuation (33, 34) du côté de la bandelette de test d'urine, de telle sorte qu'une pointe, en particulier de type canule, de l'élément d'alimentation et/ou d'évacuation (28, 29) pour l'alimentation et/ou l'évacuation de la ou d'une quantité d'urine déterminée dans ou hors de la chambre d'analyse (9) du côté de la bandelette de test d'urine, pénètre dans la zone d'alimentation et/ou d'évacuation (33, 34) du côté de la bandelette de test d'urine.

3. Dispositif selon l'une quelconque des revendications précédentes, **caractérisé par** au moins un dispositif de transport (12) qui est prévu pour le transport d'au moins une bandelette de test d'urine (10) dans une zone de détection définie du côté du dispositif, dans laquelle une détection d'une variation au moins partielle de l'au moins un paramètre détectable optiquement est possible au moyen du dispositif de détection (11), et/ou hors de la ou d'une telle zone de détection.

4. Dispositif selon la revendication 3, **caractérisé en ce que** le dispositif de transport (12) comprend ou est réalisé sous forme d'au moins un élément de transport supporté de manière rotative avec des portions de transport pour le transport d'au moins une bandelette de test d'urine (10), en particulier une roue à bossages (26) avec des portions de transport réalisées par des bossages disposés de manière espacée sur la périphérie pour le transport d'au moins une bandelette de test d'urine (10), et/ou d'au moins une bande de transport avec des portions de transport pour le transport d'au moins une bandelette de test d'urine (10) et/ou d'au moins un rouleau de transport depuis lequel un assemblage (13) de plusieurs bandelettes de test d'urine (10) connectées les unes aux autres en forme de bande ou de sangle peuvent être déroulées ou sur lequel un assemblage (13) de plusieurs bandelettes de test d'urine (10) connectées les unes aux autres en forme de bande ou de sangle peut rouler.

5. Dispositif selon la revendication 4, **caractérisé en ce que** le dispositif de transport (12) comprend au moins deux rouleaux de transport, un assemblage (13) de plusieurs bandelettes de test d'urine (10) connectées les unes aux autres en forme de bande ou de sangle pouvant être déroulé d'un premier rouleau de transport de telle sorte qu'au moins une bandelette de test d'urine (10) à transporter dans la zone de détection puisse être déplacée ou soit déplacée dans la zone de détection, et pouvant être enroulé sur un deuxième rouleau de transport de telle sorte que l'au moins une bandelette de test d'urine (10) puisse être déplacée ou soit déplacée hors de la zone de détection.

6. Dispositif selon l'une quelconque des revendications précédentes, **caractérisé par** au moins un dispositif de séparation (25) monté en aval du dispositif de détection (11), qui est prévu pour séparer au moins une bandelette de test d'urine (10) d'un assemblage (13) de plusieurs bandelettes de test d'urine (10) connectées les unes aux autres en forme de bande ou en forme de sangle.

7. Dispositif selon l'une quelconque des revendications précédentes, **caractérisé par** au moins une partie de boîtier (6) qui est réalisée pour recevoir le dispositif d'alimentation et d'évacuation (7), le dispositif de détection (11) ainsi qu'éventuellement d'autres dispositifs du dispositif (2).

8. Dispositif selon l'une quelconque des revendications précédentes, **caractérisé par** au moins une conduite de dérivation (41) qui relie une conduite d'alimentation (15) conduisant dans un élément d'alimentation (28) et une conduite d'évacuation (19) conduisant hors d'un élément d'évacuation (29), au moins un dispositif de soupape (42) pour ouvrir et fermer la conduite de dérivation (41) étant branché dans la conduite de dérivation (41).

9. Dispositif selon l'une quelconque des revendications précédentes, **caractérisé en ce que** le dispositif de détection (11) est prévu pour communiquer avec au moins un dispositif d'analyse qui est prévu pour analyser une information de détection générée par le dispositif de détection (11) et pour déterminer une information d'analyse décrivant une analyse de la quantité d'urine se trouvant sur la zone d'analyse (8) de la bandelette de test d'urine (10).

10. Dispositif selon l'une quelconque des revendications précédentes, **caractérisé par** un dispositif de commande qui est prévu pour commander le fonctionnement du dispositif d'alimentation et d'évacuation (7), du dispositif de détection (11) ainsi qu'éventuellement d'autres dispositifs du côté du dispositif, en particulier d'au moins un dispositif de détermination de pression (38), d'au moins un dispositif de pompe (37), et d'au moins un dispositif de soupape (42) branché dans une conduite de dérivation correspondante (41).

11. Système sanitaire (1) comprenant un WC (3) notamment accroché au mur ou posé sur le sol, comprenant un corps de base (4), notamment en céramique, et un dispositif (2) selon l'une quelconque des revendications 1 à 10.

12. Système sanitaire selon la revendication 11, **caractérisé par** au moins un dispositif de prélèvement (14) réalisé ou disposé dans le corps de base (4), comprenant au moins un élément tubulaire (21) disposé dans une ouverture du corps de base (4), qui est supporté de manière déplaçable entre une position d'ouverture et une position de fermeture par rapport à un élément de fermeture (22) .

13. Système sanitaire selon la revendication 12, **caractérisé en ce que** l'élément tubulaire (21) présente, sur ou dans la région de son extrémité libre opposée au corps de base (4), un moyen de raccordement pour le raccordement au, ou à un, dispositif d'alimentation et d'évacuation (7) du côté du dispositif.

14. Système sanitaire selon l'une quelconque des revendications 11 à 13, **caractérisé en ce qu'**une conduite d'évacuation (19) conduisant hors du, ou d'un, dispositif d'alimentation et d'évacuation (7) du côté du dispositif, conduit dans un espace interne limité par le corps de base (4).

15. Système sanitaire selon l'une quelconque des revendications 11 à 14, **caractérisé par** au moins un dispositif de chasse d'eau (43) pour effectuer une opération de rinçage du WC (3), au moins une conduite fluidique du côté du dispositif de chasse d'eau (17) pouvant être ou étant connectée à la, ou à une, conduite d'alimentation (15) conduisant dans un dispositif d'alimentation et d'évacuation (7) du côté du dispositif.
